# EUROPEAN PATENT APPLICATION

(11) **EP 4 640 689 A1**
(43) Date of publication of application: **29.10.2025**
(21) Application number: 23905991.8
(22) Date of filing: 20.12.2023
(51) Int. Cl.: C07D 498/22, A61K 31/553, A61P 35/00

(54) **CRYSTAL FORM OF KRAS G12D INHIBITOR AND PREPARATION METHOD THEREFOR**

(30) Priority: 20.12.2022 CN 202211642003
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: SHANG, Tingting, Lianyungang, Jiangsu 222047 (CN); YANG, Junran, Lianyungang, Jiangsu 222047 (CN); DU, Zhenxing, Lianyungang, Jiangsu 222047 (CN); ZHANG, Baolei, Shanghai 200245 (CN); XI, Zhuoxun, Shanghai 200245 (CN); FENG, Jun, Shanghai 200245 (CN)
(74) Representative: Dompatent Partnerschaft von Patent- und Rechtsanwälten mbB
(86) International application number: PCT/CN2023/140189
(87) International publication number: WO 2024/131829

(57) **Abstract**

The present disclosure relates to a crystal form of a KRAS G12D inhibitor and a preparation method therefor. Specifically, provided in the present disclosure are crystal forms A, B, C, D, E, F, G, H, I, J, K, L, M, N and O of a compound as represented by formula (I), and a preparation method therefor.

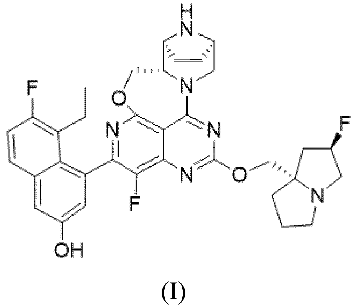

## Description

The present application claims priority to Chinese Patent Application No. 2022116420036 filed on December 20, 2022, which is incorporated herein by reference in its entirety.

### TECHNICAL FIELD

The present disclosure pertains to the technical field of pharmaceutics and relates to a crystal form of a KRAS G12D inhibitor and a preparation method therefor.

### BACKGROUND

RAS is one of the oncogenes with the highest mutation rate in tumors, and about 30% of human malignancies are associated with mutations of the RAS gene. The RAS family includes KRAS, NRAS, and HRAS, and KRAS mutations are the most common and account for approximately 85%. KRAS mutations are common in solid tumors, with high-frequency mutations in the three fatal cancers in humans: lung cancer (17%), colorectal cancer (33%), and pancreatic cancer (61%). A total of 97% of the KRAS gene mutations involve the mutation of amino acid residue 12 or 13, and G12D is an important mutation. Data analysis of Western populations shows that the G12D mutation is found in 36%, 12%, and 4% of pancreatic cancer, colorectal cancer, and non-small cell lung cancer patients, respectively.

After KRAS is activated, it regulates multiple functions such as cell proliferation, survival, migration, and metabolism through a plurality of downstream signaling pathways represented by RAF-MEK-ERK, PI3K-AKT-mTOR, and TIAM1-RAc. After the mutation of the KRAS gene, the protein is continuously activated, resulting in continuous activation of downstream signaling pathways, and thus promoting tumorigenesis.

KRAS protein has long been considered as an undruggable drug target because it lacks conventional small molecule binding sites on its surface and it is extremely difficult to inhibit due to its ultrahigh affinity for guanylic acid. However, based on the importance and prevalence of abnormal KRAS activation in cancer progression, KRAS has been and remains a target of high interest for drug development. Currently, except for KRAS G12C inhibitors, there is a lack of KRAS inhibitors that are effective against other mutations, such that most patients with KRAS mutations remain non-medicated. G12D is a mutant widely and highly expressed in multiple tumors, and it is of important clinical significance to develop inhibitors against it.

Patent application PCT/CN2022/100016 provides a novel KRAS G12D inhibitor (formula I), and the compound has relatively good pharmaceutical activity. In order to meet the needs of drug development, research on crystal forms of the compound is necessary. The crystal forms prepared in the present disclosure have good physical and chemical stability and meet the needs of drug development and storage,

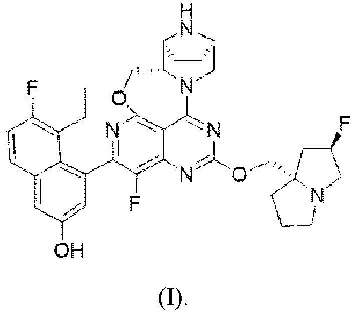

### SUMMARY

The present disclosure provides crystal forms of the compound represented by formula (I) and preparation methods therefor,

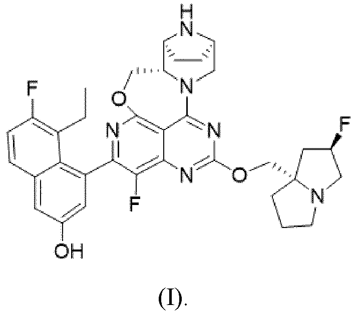

The present disclosure provides a crystal form A of the compound represented by formula (I), and an X-ray powder diffraction pattern of the crystal form has characteristic peaks at 2θ angles of 9.2, 13.1, 14.5, 16.6, 17.7, and 21.5.

In some embodiments, provided is the crystal form A of the compound represented by formula (I), and the X-ray powder diffraction pattern of the crystal form has characteristic peaks at 2θ angles of 6.6, 9.2, 10.1, 10.7, 13.1, 14.5, 15.1, 16.6, 17.7, 19.6, 20.9, 21.5, 22.2, 24.0, 24.5, 24.9, 25.9, 27.2, 28.6, and 30.2.

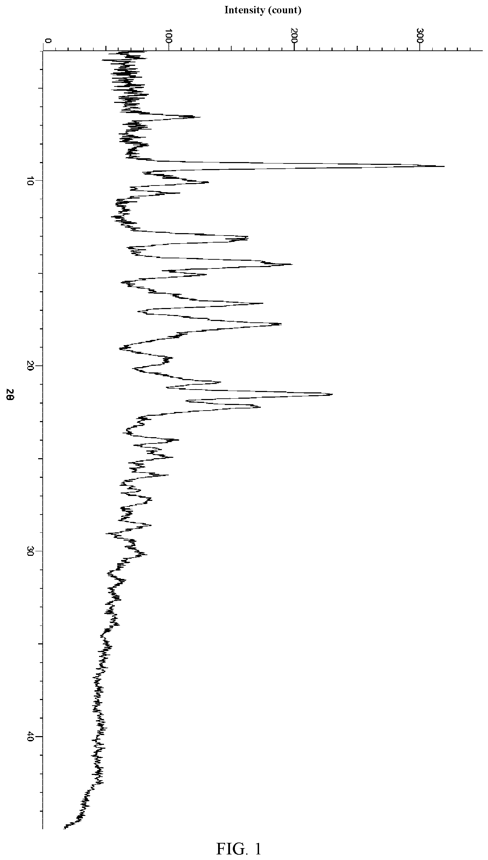

In some embodiments, provided is the crystal form A of the compound represented by formula (I), and the X-ray powder diffraction pattern of the crystal form is shown in FIG. 1.

In some embodiments, the 2θ angles for the crystal form A of the compound represented by formula (I) have a margin of error of ±0.2.

The present disclosure further provides a method for preparing the crystal form A of the compound represented by formula (I), the method comprising: mixing the compound represented by formula (I) with a proper amount of a solvent, and cooling for crystallization, wherein the solvent is selected from the group consisting of one or more of methanol and water/methanol. The present disclosure also provides a crystal form B of the compound represented by formula (I), and an X-ray powder diffraction pattern of the crystal form has characteristic peaks at 2θ angles of 8.8, 12.7, 14.6, 14.9, 16.2, 18.0, and 21.4.

In some embodiments, provided is the crystal form B of the compound represented by formula (I), and the X-ray powder diffraction pattern of the crystal form has characteristic peaks at 2θ angles of 7.3, 8.8, 10.1, 10.6, 12.7, 14.6, 14.9, 16.2, 16.6, 17.3, 18.0, 19.2, 20.0, 21.4, 22.2, 24.4, 25.0, 25.7, 28.7, and 29.4.

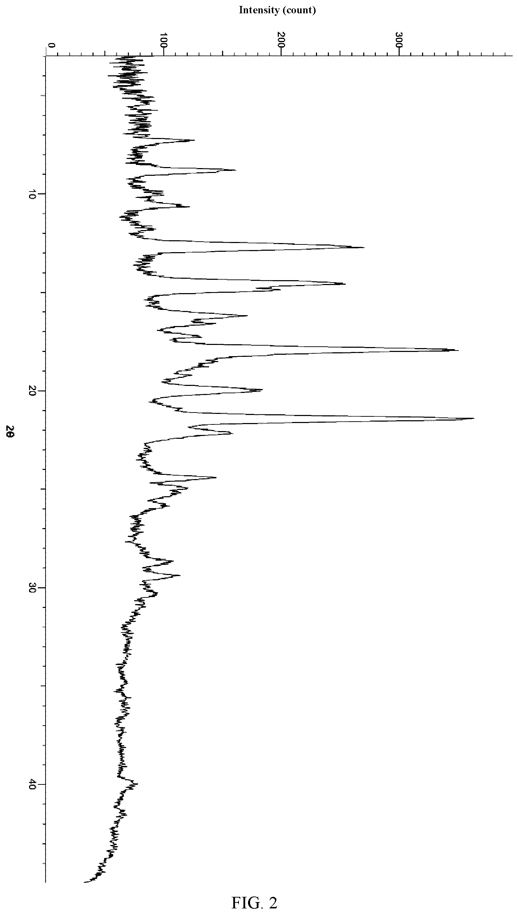

In some embodiments, provided is the crystal form B of the compound represented by formula (I), and the X-ray powder diffraction pattern of the crystal form is shown in FIG. 2.

In some embodiments, the 2θ angles for the crystal form B of the compound represented by formula (I) have a margin of error of ±0.2.

The present disclosure further provides a method for preparing the crystal form B of the compound represented by formula (I), the method comprising: mixing the compound represented by formula (I) with a proper amount of a solvent, and cooling for crystallization, wherein the solvent is selected from the group consisting of one or more of ethanol, isopropanol, n-propanol, ethyl acetate/ethanol, and ethyl acetate/n-heptane.

The present disclosure also provides a crystal form C of the compound represented by formula (I), and an X-ray powder diffraction pattern of the crystal form has characteristic peaks at 2θ angles of 5.7, 7.6, 11.1, 11.4, 17.9, and 19.3.

In some embodiments, provided is the crystal form C of the compound represented by formula (I), and the X-ray powder diffraction pattern of the crystal form has characteristic peaks at 2θ angles of 5.7, 6.4, 6.9, 7.6, 11.1, 11.4, 12.7, 13.9, 15.9, 17.2, 17.9, 19.3, 23.9, 24.4, 25.7, and 26.4.

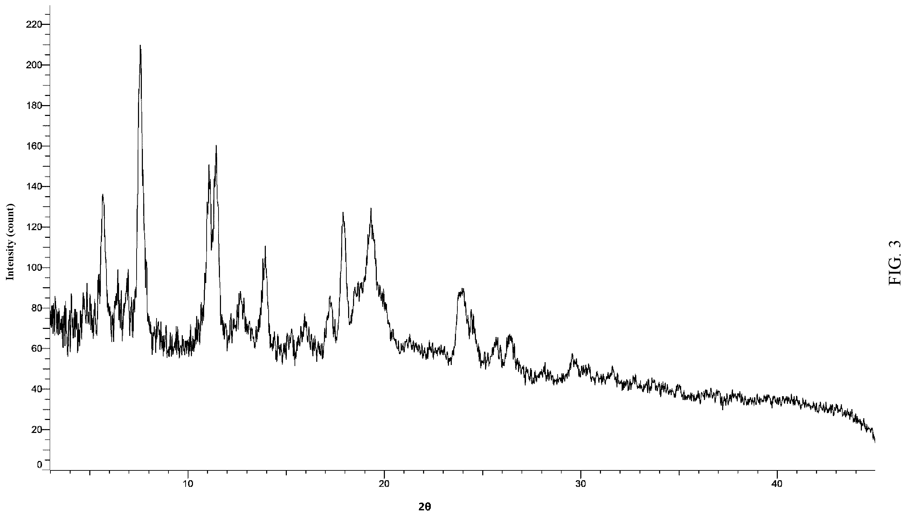

In some embodiments, provided is the crystal form C of the compound represented by formula (I), and the X-ray powder diffraction pattern of the crystal form is shown in FIG. 3.

In some embodiments, the 2θ angles for the crystal form C of the compound represented by formula (I) have a margin of error of ±0.2.

The present disclosure further provides a method for preparing the crystal form C of the compound represented by formula (I), the method comprising: mixing the compound represented by formula (I) with a proper amount of a solvent, and crystallizing, wherein the solvent is acetonitrile.

The present disclosure also provides a crystal form D of the compound represented by formula (I), and an X-ray powder diffraction pattern of the crystal form has characteristic peaks at 2θ angles of 5.8, 6.7, and 8.2.

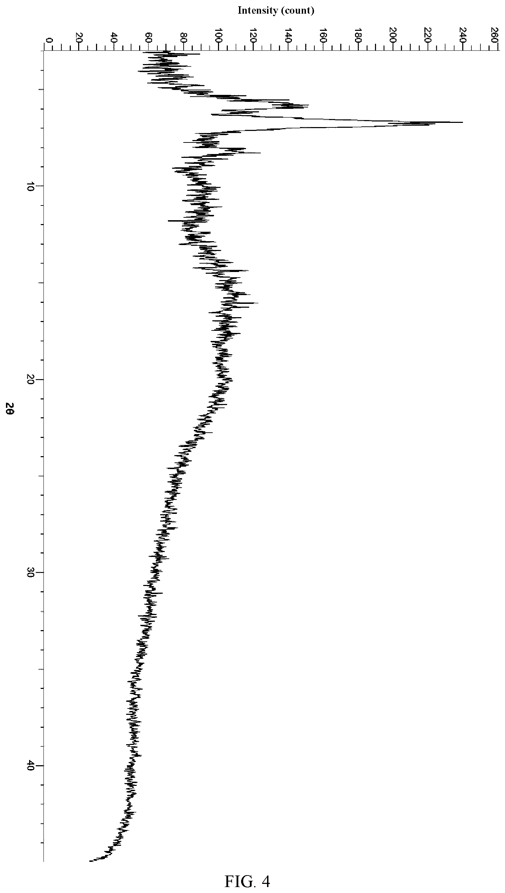

In some embodiments, provided is the crystal form D of the compound represented by formula (I), and the X-ray powder diffraction pattern of the crystal form is shown in FIG. 4.

In some embodiments, the 2θ angles for the crystal form D of the compound represented by formula (I) have a margin of error of ±0.2.

The present disclosure further provides a method for preparing the crystal form D of the compound represented by formula (I), the method comprising: mixing the compound represented by formula (I) with a proper amount of a solvent I, adding a solvent II, and stirring for crystallization, wherein the solvent I is selected from the group consisting of one or more of ethanol and acetonitrile/methanol, and the solvent II is selected from the group consisting of one or more of isopropyl acetate and methyl *tert*-butyl ether.

The present disclosure also provides a crystal form E of the compound represented by formula (I), and an X-ray powder diffraction pattern of the crystal form has characteristic peaks at 2θ angles of 6.7, 7.9, 9.4, 11.8, 12.3, 13.5, 14.0, 16.2, 17.5, 18.1, 20.3, 21.5, 23.6, and 24.9.

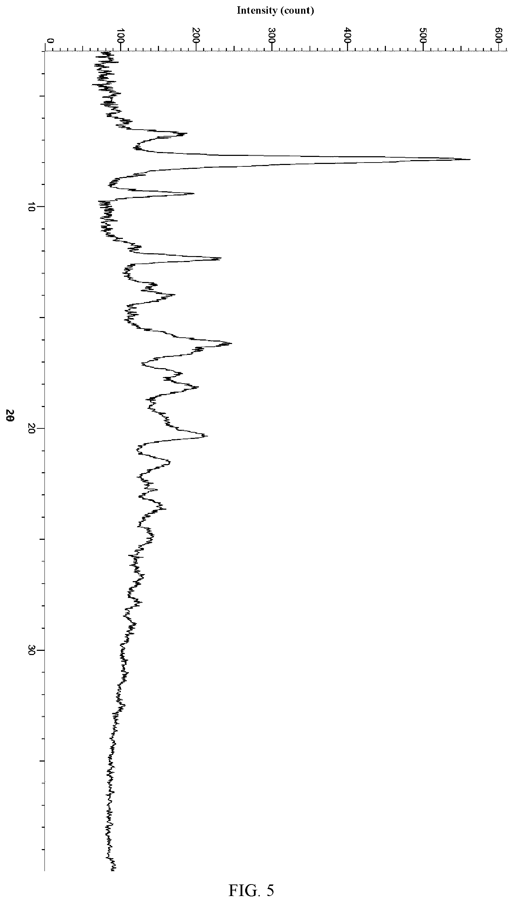

In some embodiments, provided is the crystal form E of the compound represented by formula (I), and the X-ray powder diffraction pattern of the crystal form is shown in FIG. 5.

In some embodiments, the 2θ angles for the crystal form E of the compound represented by formula (I) have a margin of error of ±0.2.

The present disclosure further provides a method for preparing the crystal form E of the compound represented by formula (I), the method comprising: mixing the compound represented by formula (I) with a proper amount of a solvent, and volatilizing for crystallization, wherein the solvent is ethyl acetate.

The present disclosure also provides a crystal form F of the compound represented by formula (I), and an X-ray powder diffraction pattern of the crystal form has characteristic peaks at 2θ angles of 5.7, 7.8, 10.4, 11.3, 12.4, 13.7, 15.6, 16.0, 17.1, 18.0, 19.0, 19.7, 23.3, 24.2, 25.0, 25.8, and 26.9.

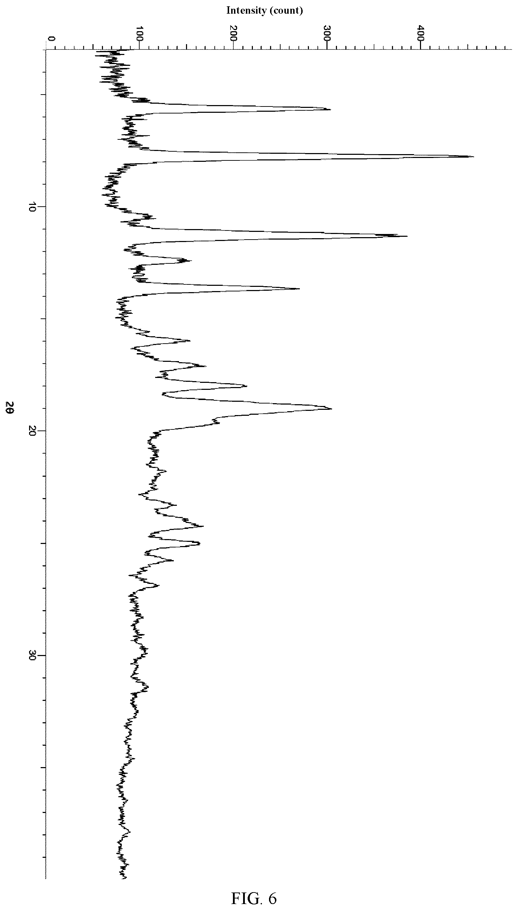

In some embodiments, provided is the crystal form F of the compound represented by formula (I), and the X-ray powder diffraction pattern of the crystal form is shown in FIG. 6.

In some embodiments, the 2θ angles for the crystal form F of the compound represented by formula (I) have a margin of error of ±0.2.

The present disclosure further provides a method for preparing the crystal form F of the compound represented by formula (I), the method comprising: mixing the compound represented by formula (I) with a proper amount of a solvent, and crystallizing, wherein the solvent is methyl tert-butyl ether.

The present disclosure also provides a crystal form G of the compound represented by formula (I), and an X-ray powder diffraction pattern of the crystal form has characteristic peaks at 2θ angles of 7.7, 10.9, 12.8, 15.4, 20.5, and 22.0.

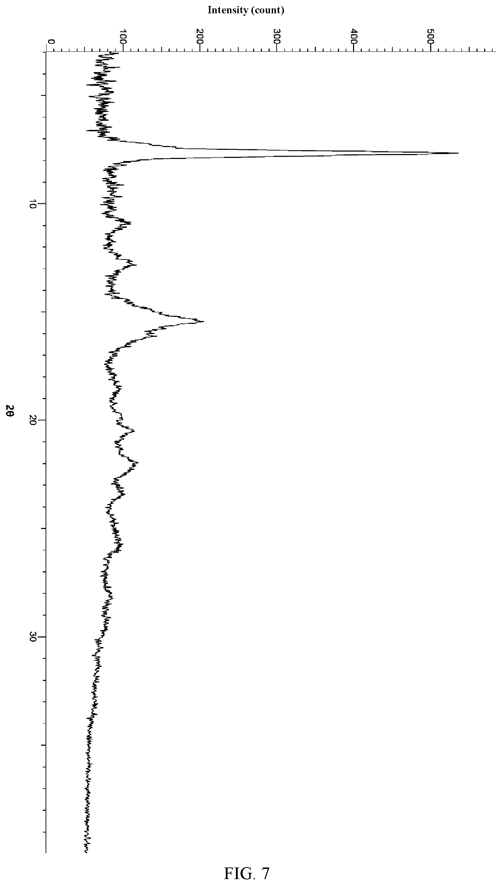

In some embodiments, provided is the crystal form G of the compound represented by formula (I), and the X-ray powder diffraction pattern of the crystal form is shown in FIG. 7.

In some embodiments, the 2θ angles for the crystal form G of the compound represented by formula (I) have a margin of error of ±0.2.

The present disclosure further provides a method for preparing the crystal form G of the compound represented by formula (I), the method comprising: mixing the compound represented by formula (I) with a proper amount of a solvent, and stirring at room temperature for two days for crystallization, wherein the solvent is dichloromethane.

The present disclosure also provides a crystal form H of the compound represented by formula (I), and an X-ray powder diffraction pattern of the crystal form has characteristic peaks at 2θ angles of 7.5, 8.6, 9.4, 12.3, 14.0, 15.9, 17.4, 18.4, 19.4, 20.5, 21.6, 22.9, 24.8, 26.6, and 27.2.

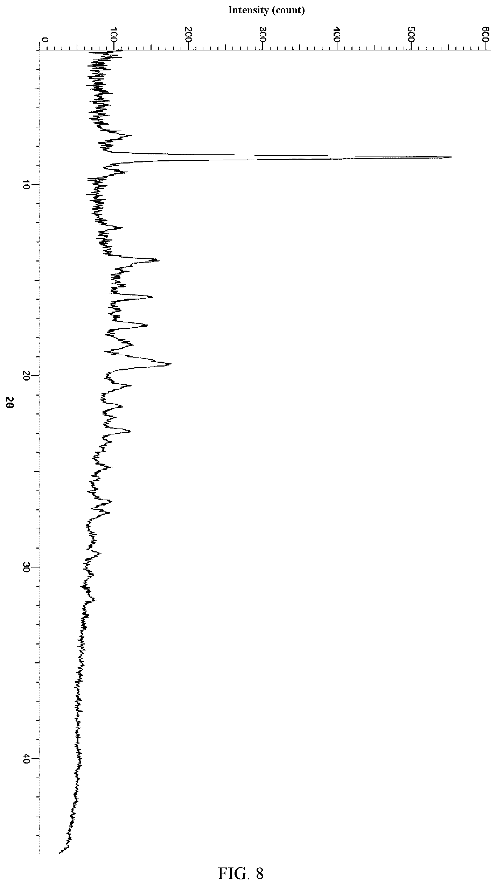

In some embodiments, provided is the crystal form H of the compound represented by formula (I), and the X-ray powder diffraction pattern of the crystal form is shown in FIG. 8.

In some embodiments, the 2θ angles for the crystal form H of the compound represented by formula (I) have a margin of error of ±0.2.

The present disclosure further provides a method for preparing the crystal form H of the compound represented by formula (I), the method comprising: heating the crystal form E of the compound represented by formula (I).

The present disclosure also provides a crystal form I of the compound represented by formula (I), and an X-ray powder diffraction pattern of the crystal form has characteristic peaks at 2θ angles of 6.9, 15.3, 16.6, and 20.1.

In some embodiments, provided is the crystal form I of the compound represented by formula (I), and the X-ray powder diffraction pattern of the crystal form has characteristic peaks at 2θ angles of 5.6, 6.9, 8.3, 9.6, 11.4, 13.9, 15.3, 16.6, 18.4, 20.1, 20.6, 21.1, 22.1, 23.1, 24.5, 26.0, and 33.0.

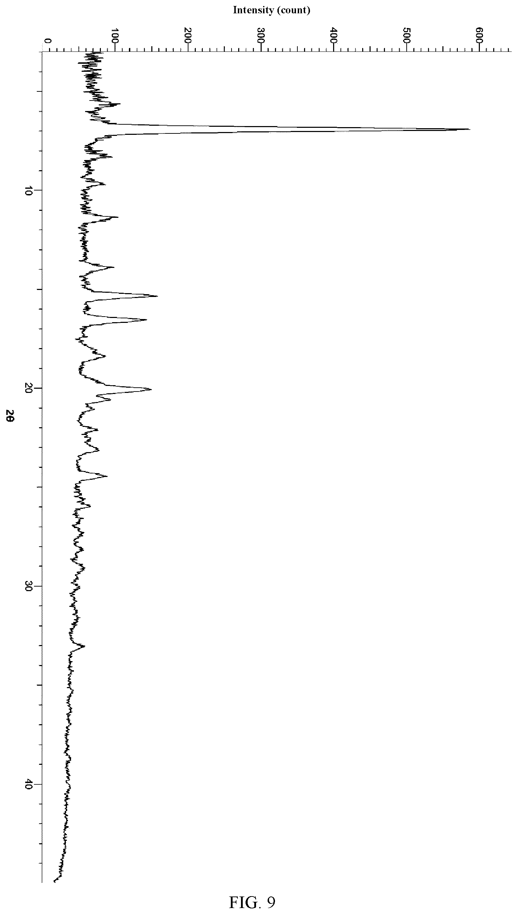

In some embodiments, provided is the crystal form I of the compound represented by formula (I), and the X-ray powder diffraction pattern of the crystal form is shown in FIG. 9.

In some embodiments, the 2θ angles for the crystal form I of the compound represented by formula (I) have a margin of error of ±0.2.

The present disclosure further provides a method for preparing the crystal form I of the compound represented by formula (I), the method comprising: mixing the compound represented by formula (I) with a proper amount of a solvent, and stirring at room temperature for crystallization, wherein the solvent is selected from the group consisting of one or more of methanol, ethanol, *n*-propanol, water/methanol, water/ethanol, water/isopropanol, acetonitrile/methanol, acetone, water/acetone, 2-butanone, acetonitrile, ethyl acetate, isopropyl acetate, *n*-heptane, tetrahydrofuran/ethanol, ethyl acetate/ethanol, ethyl acetate/*n*-heptane, isopropyl ether, and methyl *tert*-butyl ether.

The present disclosure also provides a crystal form J of the compound represented by formula (I), and an X-ray powder diffraction pattern of the crystal form has characteristic peaks at 2θ angles of 7.2, 7.7, 8.5, 9.8, 10.6, and 14.9.

In some embodiments, provided is the crystal form J of the compound represented by formula (I), and the X-ray powder diffraction pattern of the crystal form has characteristic peaks at 2θ angles of 7.2, 7.7, 8.5, 9.8, 10.6, 12.2, 13.5, 14.9, 15.7, 17.1, 17.9, 19.1, 20.0, 20.5, 21.6, 23.3, 24.3, 25.1, 25.7, and 28.3.

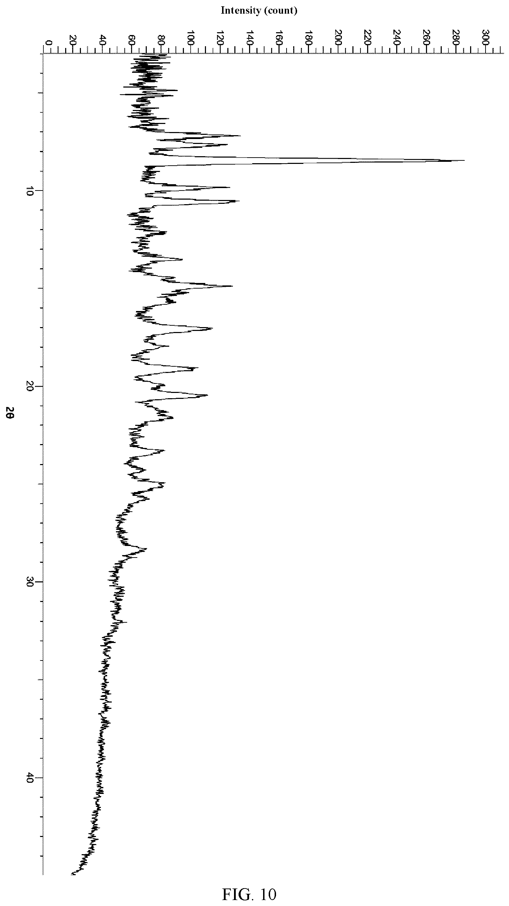

In some embodiments, provided is the crystal form J of the compound represented by formula (I), and the X-ray powder diffraction pattern of the crystal form is shown in FIG. 10.

In some embodiments, the 2θ angles for the crystal form J of the compound represented by formula (I) have a margin of error of ±0.2.

The present disclosure further provides a method for preparing the crystal form J of the compound represented by formula (I), the method comprising: mixing the compound represented by formula (I) with a proper amount of a solvent, and stirring at room temperature overnight for crystallization, wherein the solvent is dichloromethane.

The present disclosure also provides a crystal form K of the compound represented by formula (I), and an X-ray powder diffraction pattern of the crystal form has characteristic peaks at 2θ angles of 5.3, 6.7, 10.7, 12.3, 13.5, 14.8, 18.1, 20.6, 21.3, and 27.3.

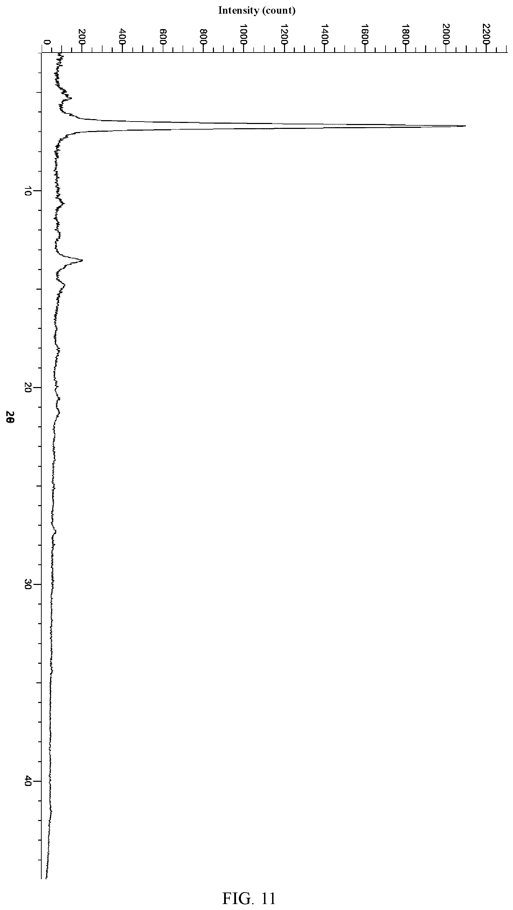

In some embodiments, provided is the crystal form K of the compound represented by formula (I), and the X-ray powder diffraction pattern of the crystal form is shown in FIG. 11.

In some embodiments, the 2θ angles for the crystal form K of the compound represented by formula (I) have a margin of error of ±0.2.

The present disclosure further provides a method for preparing the crystal form K of the compound represented by formula (I), the method comprising: mixing the compound represented by formula (I) with a proper amount of a solvent, and crystallizing, wherein the solvent is tetrahydrofuran.

The present disclosure also provides a crystal form L of the compound represented by formula (I), and an X-ray powder diffraction pattern of the crystal form has characteristic peaks at 2θ angles of 6.8, 8.4, 9.5, 12.3, 14.0, 16.8, 18.3, 20.3, 21.7, 22.7, and 24.8.

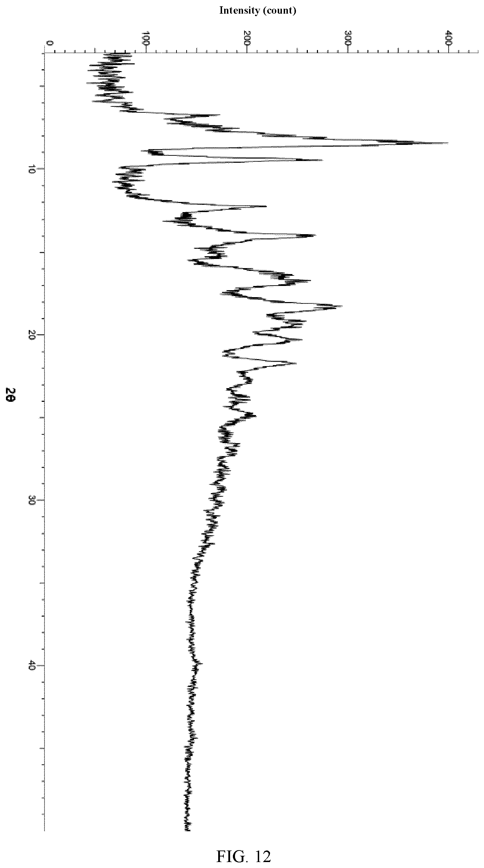

In some embodiments, provided is the crystal form L of the compound represented by formula (I), and the X-ray powder diffraction pattern of the crystal form is shown in FIG. 12.

In some embodiments, the 2θ angles for the crystal form L of the compound represented by formula (I) have a margin of error of ±0.2.

The present disclosure further provides a method for preparing the crystal form L of the compound represented by formula (I), the method comprising: mixing the compound represented by formula (I) with a proper amount of a solvent, and crystallizing, wherein the solvent is ethyl acetate.

The present disclosure also provides a crystal form M of the compound represented by formula (I), and an X-ray powder diffraction pattern of the crystal form has characteristic peaks at 2θ angles of 6.7, 7.8, 9.5, 11.9, 12.3, 13.0, 13.6, 14.3, 14.9, 15.8, 16.8, 17.7, 18.3, 19.1, 20.4, 21.7, 22.4, 23.9, 24.9, 26.7, 27.4, and 27.9.

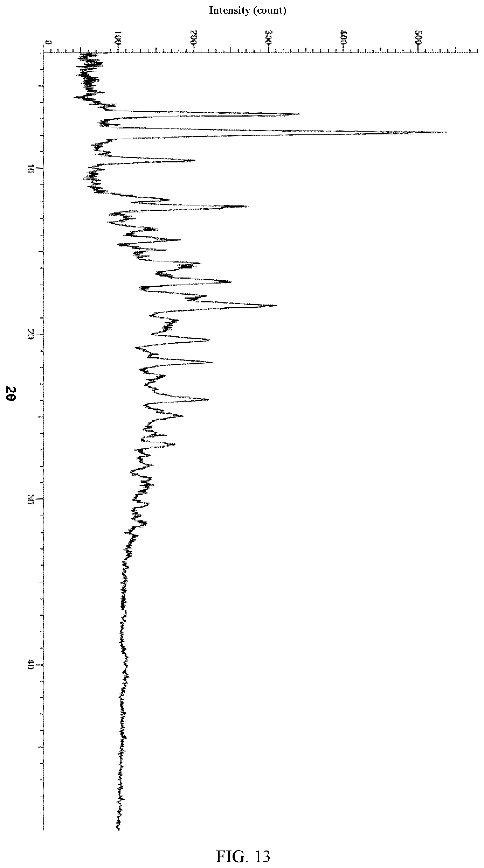

In some embodiments, provided is the crystal form M of the compound represented by formula (I), and the X-ray powder diffraction pattern of the crystal form is shown in FIG. 13.

In some embodiments, the 2θ angles for the crystal form M of the compound represented by formula (I) have a margin of error of ±0.2.

The present disclosure further provides a method for preparing the crystal form M of the compound represented by formula (I), the method comprising: mixing the compound represented by formula (I) with a proper amount of a solvent, and crystallizing, wherein the solvent is isopropyl acetate.

The present disclosure also provides a crystal form N of the compound represented by formula (I), and an X-ray powder diffraction pattern of the crystal form has characteristic peaks at 2θ angles of 6.6, 7.7, 8.2, 9.1, 10.1, 11.3, 11.8, 12.6, 14.0, 15.4, 17.8, 19.0, 19.9, 21.7, 23.1, 24.0, and 25.6.

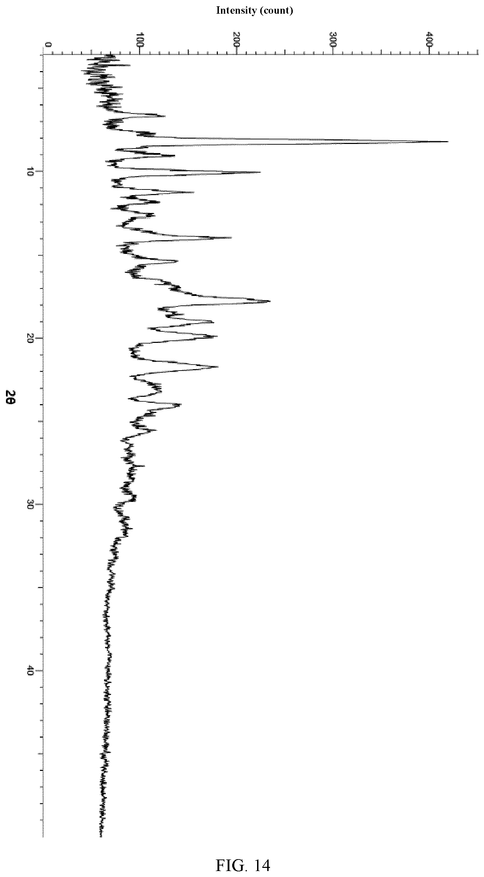

In some embodiments, provided is the crystal form N of the compound represented by formula (I), and the X-ray powder diffraction pattern of the crystal form is shown in FIG. 14.

In some embodiments, the 2θ angles for the crystal form N of the compound represented by formula (I) have a margin of error of ±0.2.

The present disclosure further provides a method for preparing the crystal form N of the compound represented by formula (I), the method comprising: mixing the compound represented by formula (I) with a proper amount of a solvent, and crystallizing, wherein the solvent is acetone.

The present disclosure also provides a crystal form O of the compound represented by formula (I), and an X-ray powder diffraction pattern of the crystal form has characteristic peaks at 2θ angles of 7.0, 7.5, 8.9, 9.7, 10.5, 12.0, 12.8, 13.2, 14.0, 14.4, 15.1, 17.5, 18.1, 18.8, 19.6, 20.6, 21.2, 22.3, 24.2, 25.0, 25.9, 27.7, and 28.3.

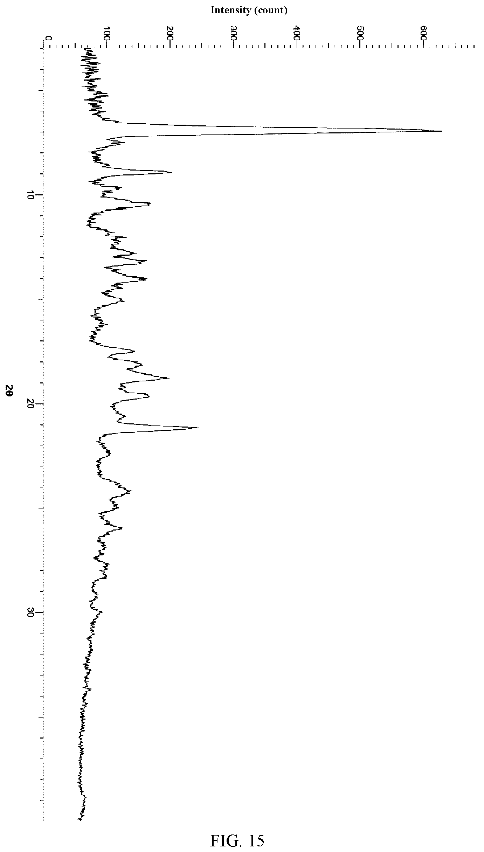

In some embodiments, provided is the crystal form O of the compound represented by formula (I), and the X-ray powder diffraction pattern of the crystal form is shown in FIG. 15.

In some embodiments, the 2θ angles for the crystal form O of the compound represented by formula (I) have a margin of error of ±0.2.

The present disclosure further provides a method for preparing the crystal form O of the compound represented by formula (I), the method comprising: mixing the compound represented by formula (I) with a proper amount of a solvent, and stirring at room temperature for crystallization, wherein the solvent is isopropanol.

The crystal forms obtained by the present disclosure are structurally analyzed and studied using X-ray powder diffraction patterns (XRPD) and differential scanning calorimetry (DSC).

The crystallization methods for the crystal forms in the present disclosure are conventional, such as volatilizing for crystallization, cooling for crystallization, or room-temperature crystallization. The starting materials used in the preparation methods for the salts or crystal forms of the present disclosure may be any form of the compound represented by formula (I); specific forms include, but are not limited to an amorphous form, any crystal form, a hydrate, a solvate, etc.

The present disclosure further provides a pharmaceutical composition comprising the following components: (a) a crystal form of the compound represented by formula (I); and (b) an optional pharmaceutically acceptable carrier, diluent, or excipient.

The present disclosure also provides a preparation method for a pharmaceutical composition, the method comprising a step of: mixing (a) a crystal form of the compound represented by formula (I) with (b) an optional pharmaceutically acceptable carrier, diluent, or excipient.

The present disclosure further provides use of the aforementioned crystal forms of the compound represented by formula (I) or the aforementioned composition in the manufacture of a medicament for inhibiting KRAS G12D.

The present disclosure further provides use of the aforementioned crystal forms of the compound represented by formula (I) or the aforementioned composition in the manufacture of a medicament for treating and/or preventing a disease or disorder, wherein the disease or disorder is a cancer.

In some embodiments, the disease or disorder is selected from the group consisting of brain cancer, thyroid cancer, head and neck cancer, nasopharyngeal carcinoma, laryngeal cancer, oral cancer, salivary gland cancer, esophageal cancer, gastric cancer, lung cancer, liver cancer, renal cancer, pancreatic cancer, gallbladder cancer, cholangiocarcinoma, colorectal cancer, small intestine cancer, gastrointestinal stromal tumor, urothelial carcinoma, urethral cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, neuroglioma, neuroblastoma, and glioblastoma.

In some embodiments, the disease or disorder is selected from the group consisting of pancreatic cancer, colorectal cancer, and non-small cell lung cancer.

In the specification and the claims of the present application, unless otherwise specified, the scientific and technological terms used herein have the meanings generally understood by those skilled in the art. However, definitions and explanations for some of the related terms are provided below for a better understanding of the present disclosure. In addition, if the definitions and explanations of the terms provided herein are not consistent with the meanings generally understood by those skilled in the art, the definitions and explanations of the terms provided herein shall prevail.

As used herein, "X-ray powder diffraction pattern" or "XRPD" refers to a set of X-ray powder diffraction peaks obtained according to the Bragg's equation 2d sinθ = nλ (in the equation, λ is the wavelength of X-ray, the diffraction order n is any positive integer, and the first-order diffraction peak is generally taken, i.e., n = 1). When X-ray is incident on a certain atomic plane with the d-lattice plane spacing of a crystal or a partial crystal sample at a grazing angle θ (the complementary angle of incidence angle, also known as Bragg angle), the Bragg's equation can be satisfied.

As used herein, "X-ray powder diffraction pattern" or "XRPD" refers to a pattern obtained by using Cu-Kα radiation in an X-ray powder diffractometer.

As used herein, "differential scanning calorimetry" or "DSC" refers to measurement of the temperature difference and heat flow difference between a sample and a reference substance during a ramping or thermostatic process to characterize all the physical changes and chemical changes related to the thermal effect, and to obtain the phase change information of the sample. As used herein, "thermogravimetric analysis" or "TGA" refers to continuous measurement of the mass change of a sample as a function of temperature or time at a programmed temperature.

As used herein, "2θ" or "2θ angle" refers to a diffraction angle; θ is the Bragg angle in unit ° or degree, and the 2θ angles have a margin of error of ±0.3, ±0.2, or ±0.1.

As used herein, "interplanar spacing (d)" refers to 3 selected non-parallel unit vectors (a, b, and c) in the space lattice, each of which connects two adjacent lattice points and all of which divide the lattice into juxtaposed parallelepiped units. The space lattice is divided according to the connecting lines of the determined parallelepiped units, giving a set of linear grids called space lattice or crystal lattice. The lattice and the crystal lattice reflect the periodicity of the crystal structure by using geometrical points and lines, respectively. The interplanar spacing (the distance between two adjacent parallel crystal planes) for different crystal planes is different; the unit is Å or Angstrom.

### BRIEF DESCRIPTION OF THE DRAWINGS

FIG. 1 shows an XRPD pattern of the crystal form A of the compound represented by formula (I);
FIG. 2 shows an XRPD pattern of the crystal form B of the compound represented by formula (I);
FIG. 3 shows an XRPD pattern of the crystal form C of the compound represented by formula (I);
FIG. 4 shows an XRPD pattern of the crystal form D of the compound represented by formula (I);
FIG. 5 shows an XRPD pattern of the crystal form E of the compound represented by formula (I);
FIG. 6 shows an XRPD pattern of the crystal form F of the compound represented by formula (I);
FIG. 7 shows an XRPD pattern of the crystal form G of the compound represented by formula (I);
FIG. 8 shows an XRPD pattern of the crystal form H of the compound represented by formula (I);
FIG. 9 shows an XRPD pattern of the crystal form I of the compound represented by formula (I);
FIG. 10 shows an XRPD pattern of the crystal form J of the compound represented by formula (I);
FIG. 11 shows an XRPD pattern of the crystal form K of the compound represented by formula (I);
FIG. 12 shows an XRPD pattern of the crystal form L of the compound represented by formula (I);
FIG. 13 shows an XRPD pattern of the crystal form M of the compound represented by formula (I);
FIG. 14 shows an XRPD pattern of the crystal form N of the compound represented by formula (I);
FIG. 15 shows an XRPD pattern of the crystal form O of the compound represented by formula (I);
FIG. 16 shows an XRPD pattern of an amorphous form of the compound represented by formula (I).

### DETAILED DESCRIPTION

The present disclosure is explained below in more details with reference to examples. The examples of the present disclosure are only illustrative of the technical solutions of the present disclosure and do not limit the essence and scope of the present disclosure.

The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument or Bruker AVANCE NEO 500M, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and

THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high-pressure liquid chromatographs.

The chiral HPLC analyses were performed using an Agilent 1260 DAD high performance liquid chromatograph.

The preparative high performance liquid chromatography used Waters 2545-2767, Waters 2767-SQ Detecor2, Shimadzu LC-20AP, and Gilson GX-281 preparative chromatographs.

The preparative chiral chromatography used a Shimadzu LC-20AP preparative chromatograph. The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

The silica gel column chromatography generally used 200-300 mesh Yantai Huanghai silica gel as the carrier.

The kinase mean inhibition rates and IC₅₀ values were measured using a NovoStar microplate reader (BMG, Germany).

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling. The microwave reactions were performed using a CEM Discover-S 908860 microwave reactor. In the examples, the solutions were aqueous solutions unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvents used in the reactions, the eluent systems used in the column chromatography purification of the compounds, and the developing solvent systems used in the thin-layer chromatography include: A: a dichloromethane/methanol system, and B: *n*-hexane/ethyl acetate. The volume ratio of the solvents was adjusted depending on the polarity of the compounds, or by adding a small amount of basic or acidic reagents such as triethylamine and acetic acid.

When the compounds in the examples contained two or more chiral centers, the relative stereochemistry of these compounds was identified by NMR studies and/or X-ray diffraction. In these cases, the compounds were identified using the prefix "rel" followed by the R/S nomenclature, where R/S provides only relative stereochemical information and does not indicate absolute stereochemistry. For example, 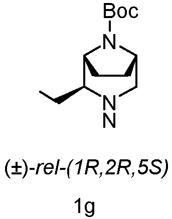 represents a 1:1 mixture of 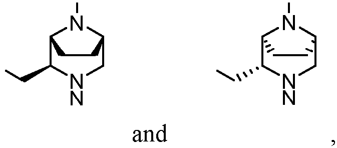 i.e., a racemate.

XRPD refers to X-ray powder diffraction: The measurement was performed using a BRUKER D8 X-ray diffractometer; specific acquisition information: a Cu anode (40 kV, 40 mA), Cu-Kα1 ray (λ = 1.54060 Å), Kα2 ray (λ = 1.54439 Å), and Kβ ray (λ = 1.39222 Å). Scan mode: θ/2θ, scan range (2θ range): 3-45°.

DSC refers to differential scanning calorimetry: The measurement was performed using a METTLER TOLEDO DSC 3+ differential scanning calorimeter, with a temperature ramp rate of 10 °C/min, specific temperature ranges shown in corresponding patterns (mostly 25-300 °C or 25-350 °C), and a nitrogen purge speed of 50 mL/min.

TGA refers to thermogravimetric analysis: The measurement was performed using a METTLER TOLEDO TGA 2 thermogravimetric analyzer, with a temperature ramp rate of 10 °C/min, specific temperature ranges shown in corresponding patterns (mostly 25-400 °C), and a nitrogen purge speed of 50 mL/min.

DVS refers to dynamic vapor sorption: The measurement was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criterion was dm/dt being not greater than 0.002%.

### Example 1

### tert-Butyl (±)-rel-(1R,2R,5S)-2-(((tert-butyldimethylsilyl)oxy)methyl)-3,8-diazabicyclo[3.2.1]octane-8-carboxylate 1h

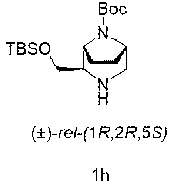

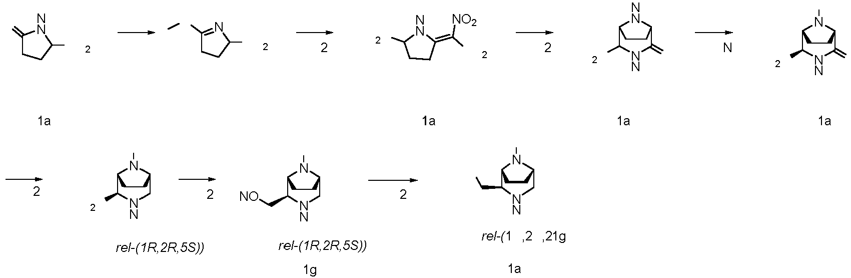

### Step 1

### Methyl (±)-5-methoxy-3,4-dihydro-2H-pyrrole-2-carboxylate 1b

Methyl (±)-2-pyrrolidone-5-formate **1a** (100 g, 698.61 mmol, Shanghai Bide) and dimethyl sulfate (110 g, 872.10 mmol) were mixed and reacted at 60 °C for 16 h, and the reaction mixture was cooled to room temperature and poured into a solution of triethylamine (100 g) and methyl tert-butyl ether (150 mL) in an ice bath. The resulting mixture was extracted with methyl *tert-*butyl ether (300 mL × 6) and then concentrated under reduced pressure to give crude title compound **1b** (90 g, yield: 81.9%). The product was directly used in the next step without purification.

MS m/z (ESI): 158.1[M+1].

### Step 2

### Methyl (±)-5-(2-methoxy-1-nitro-2-oxoethylidene)pyrrolidine-2-carboxylate 1c

Crude compound **1b** (90 g, 572.64 mmol) and methyl nitroacetate (68.18 g, 572.63 mmol) were mixed, heated to 60 °C, and stirred for reaction for 30 h. The reaction mixture was cooled to room temperature, and ethyl acetate (300 mL) was then added. The resulting mixture was stirred for 0.5 h and then filtered, and the filter cake was dried to give title compound **1c** (70 g, yield: 50%). The product was directly used in the next step without purification.

MS m/z (ESI): 245.1[M+1].

### Step 3

Methyl 4-oxo-3,8-diazabicyclo[3.2.1]octane-2-carboxylate (diastereomeric mixture) **1d** Crude compound **1c** (14 g, 57.3 mmol) was dissolved in 600 mL of methanol, and a 10% palladium on carbon catalyst (wet) (14 g) was added. The system was purged with hydrogen three times and stirred for reaction for 48 h. The reaction mixture was filtered through diatomaceous earth, and the filtrate was concentrated to give crude title compound **1d** (10 g, yield: 94.6%). The product was directly used in the next step without purification.

MS m/z (ESI): 185.2[M+1].

### Step 4

### 8-(tert-Butyl) 2-methyl (+)-rel-(1R,2R,5S)-4-oxo-3,8-diazabicyclo[3.2.1]octane-2,8-diformate 1e

Crude compound **1d** (10 g, 54.2 mmol) was dissolved in 300 mL of dichloromethane, and triethylamine (16 g, 158.12 mmol) and di-*tert*-butyl dicarbonate (11 g, 50.4 mmol, Shanghai Accela) were added in an ice bath. The reaction mixture was stirred for reaction for 14 h and concentrated under reduced pressure, and the residue was purified by silica gel column chromatography with eluent system B to give title compound **1e** (3.3 g, yield: 21.3%).

MS m/z (ESI): 285.2 [M+1].

HPLC analysis: retention time: 1.02 min; purity: 98.5% (chromatography column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

### Step 5

### 8-(tert-Butyl) 2-methyl (+)-rel-(1R,2R,5S)-3,8-diazabicyclo[3.2.1]octane-2,8-diformate 1f

Compound **1e** (400 mg, 1.4 mmol) was dissolved in 2 mL of tetrahydrofuran, and 3.5 mL of a 2 M solution of borane dimethylsulfide complex in tetrahydrofuran was added. The reaction mixture was stirred for reaction for 14 h, quenched with methanol, reacted at 50 °C for another 14 h, and concentrated under reduced pressure, and the residue was purified by silica gel column chromatography with eluent system A to give title compound **1f** (176 mg, yield: 46.2%).

MS m/z (ESI): 271.2[M+1].

### Step 6

### tert-Butyl (±)-rel-(1R,2R,5S)-2-(hydroxymethyl)-3,8-diazabicyclo[3.2.1]octane-8-carboxylate 1g

Compound **1f** (1 g, 3.69 µmol) was dissolved in 15 mL of tetrahydrofuran, and 4.4 mL of a 1 M solution of lithium aluminum hydride in tetrahydrofuran was added. The reaction mixture was stirred for reaction at 0 °C for 1 h. 0.2 mL of water, 0.2 mL of a 15% aqueous sodium hydroxide solution, and 0.4 mL of water were sequentially added, and anhydrous sodium sulfate was added. The resulting mixture was stirred for 10 min and filtered, and the filtrate was concentrated to give title compound **1g** (430 mg, yield: 47.9%). The product was directly used in the next step without purification.

MS m/z (ESI): 243.1[M+1].

### Step 7

### tert-Butyl (±)-rel-(1R,2R,5S)-2-(((tert-butyldimethylsilyl)oxy)methyl)-3,8-diazabicyclo[3.2.1]octane-8-carboxylate 1h

**1g** (8.8 g, 36.3 mmol), *tert-*butyldimethylsilyl chloride (16 g, 106.1558 mmol), and 4-dimethylaminopyridine (4 g, 32.4739 mmol) were dissolved in 200 mL of dichloromethane, and triethylamine (15 g, 148.23 mmol, 21.4286 mL) was added. The reaction mixture was stirred for reaction for 16 h and concentrated under reduced pressure, and the residue was purified by silica gel column chromatography with eluent system B to give title compound **1h** (8 g, yield: 61.7%). MS m/z (ESI): 357.1[M+1].

### Example 2

### 5-Ethyl-6-fluoro-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 1-p1

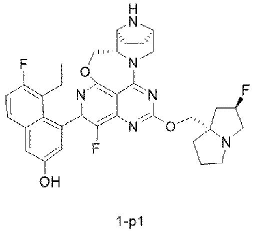

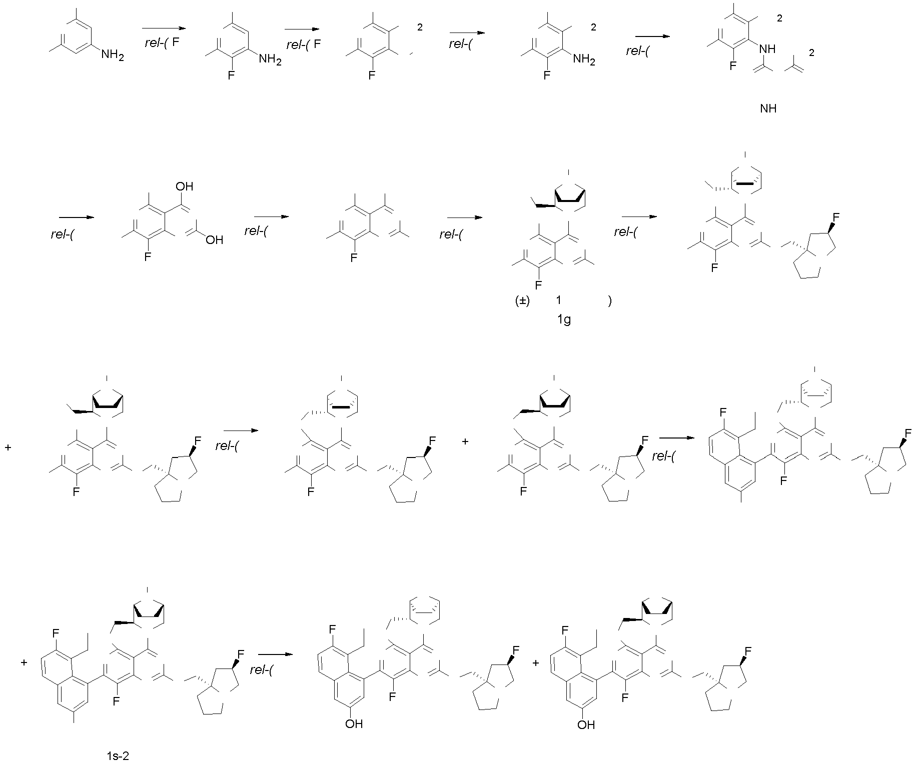

### Step 8

### 2,6-Dichloro-3-fluoropyridin-4-amine 1j

Compound 4-amino-2,6-dichloropyridine **1i** (5 g, 30.6 mmol, Shanghai Bide) was dissolved in 20 mL of *N*,*N-*dimethylformamide and 20 mL of acetonitrile, and 1-chloromethyl-4-fluoro-1,4-diazabicyclo[2.2.2]octane bis(tetrafluoroborate) (13 g, 36.8 mmol) was added. The reaction mixture was reacted at 80 °C for 0.5 h and concentrated under reduced pressure, and the residue was purified by silica gel column chromatography with eluent system B to give title compound **1j** (2.2 g, yield: 39.6%).

### Step 9

### tert-Butyl 4-((tert-butoxycarbonyl)amino)-2,6-dichloro-5-fluoronicotinate 1k

Compound **1j** (1.8 g, 9.94 mmol) was dissolved in tetrahydrofuran (50 mL), and 20 mL of a 2 M solution of sodium bis(trimethylsilyl)amide in tetrahydrofuran was added in an ice bath. The mixture was stirred for reaction for 0.5 h, and di-*tert*-butyl dicarbonate (6.5 g, 29.7 mmol) was then added. The reaction mixture was stirred for reaction for 14 h, quenched with a saturated aqueous ammonium chloride solution, and extracted with ethyl acetate (50 mL × 3). The organic phases were combined, dried over anhydrous sodium sulfate, and filtered to remove the drying agent. Then the filtrate was concentrated under reduced pressure, and the residue was purified with eluent system B to give title compound **1k** (1 g, yield: 26.3%). The product was directly used in the next step without purification.

MS m/z (ESI): 381.1[M+1].

### Step 10

### tert-Butyl 4-amino-2,6-dichloro-5-fluoronicotinate 11

Compound **1k** (1 g, 2.62 mmol) was dissolved in ethyl acetate (8 mL), and 3 mL of a 4 M solution of hydrochloric acid in dioxane was added. The mixture was stirred for reaction for 2 h, made neutral with a 4 M aqueous sodium hydroxide solution in an ice bath, and extracted with ethyl acetate (10 mL × 3). The organic phases were combined, dried over anhydrous sodium sulfate, and filtered to remove the drying agent. Then the filtrate was concentrated under reduced pressure, and the residue was purified with eluent system B to give crude title compound **11** (500 mg, yield: 67.8%).

MS m/z (ESI): 281.1[M+1].

### Step 11

### tert-Butyl 2,6-dichloro-5-fluoro-4-(3-(2,2,2-trichloroacetyl)ureido)nicotinate 1m

Crude compound **11** (500 mg, 1.77 mmol) was dissolved in tetrahydrofuran (10 mL), and trichloroacetyl isocyanate (670 mg, 3.55 mmol) was added. The reaction mixture was stirred for reaction for 30 min and concentrated under reduced pressure to give crude title compound **1m** (835 mg, yield: 99.7%). The product was directly used in the next step without purification.

MS m/z (ESI): 467.9[M+1].

### Step 12

### 5,7-Dichloro-8-fluoro-pyrido[4,3-d]pyrimidine-2,4-diol 1n

Crude compound **1m** (835 mg, 1.77 mmol) was dissolved in a 7 M solution of ammonia in methanol (10 mL). The reaction mixture was stirred for reaction for 1 h and concentrated under reduced pressure, and methyl *tert*-butyl ether (10 mL) was added to the residue. After 0.5 h of stirring, the mixture was filtered, and the filter cake was dried to give crude title compound **1n** (400 mg, yield: 89.9%). The product was directly used in the next step without purification.

MS m/z (ESI): 249.9[M+1].

### Step 13

### 2,4,5,7-Tetrachloro-8-fluoro-pyrido[4,3-d]pyrimidine 1o

Crude compound **1n** (300 mg, 1.19 mmol) was dissolved in phosphorus oxychloride (6 mL), and *N*,*N*-diisopropylethylamine (800 mg, 6.19 mmol) was added. The reaction mixture was stirred for reaction at 110 °C for 3 h, cooled to room temperature, and then concentrated under reduced pressure to give crude title compound **1o** (344 mg, yield: 97.7%). The product was directly used in the next step without purification.

MS m/z (ESI): 285.8[M+1].

### Step 14

### tert-Butyl (±)-rel-(1R,2R,5S)-2-(((tert-butyldimethylsilyl)oxy)methyl)-3-(2,5,7-trichloro-8-fluoropyrido[4,3-d]pyrimidin-4-yl)-3,8-diazabicyclo[3.2.1]octane-8-carboxylate 1p

Compound **1o** (1.0 g, 3.48 mmol) and *N*,*N*-diisopropylethylamine (0.9 g, 6.9 mmol) were dissolved in 15 mL of dichloromethane, and **1h** (1.25 g, 3.5 mmol) was added at -78 °C. The reaction mixture was stirred for reaction at that temperature for 1 h, then reacted at room temperature for 16 h, and concentrated under reduced pressure, and the residue was purified with eluent system B to give crude title compound **1p** (1.56 g, yield: 73.7%).

MS m/z (ESI): 606.2[M+1].

### Step 15

### Diastereomeric mixture of tert-butyl (1S,2S,5R)-2-(((tert-butyldimethylsilyl)oxy)methyl)-3-(5,7-dichloro-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carboxylate 1q-1 and tert-butyl (1R,2R,5S)-2-(((tert-butyldimethylsilyl)oxy)methyl)-3-(5,7-dichloro-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carboxylate 1q-2

Compound **1p** (1.4 g, 2.3 mmol) was dissolved in 1,4-dioxane (20 mL), and ((2*R*,7*aS*)-2-fluorotetrahydro-1*H*-pyrrolizin-7*a*(5*H*)-yl)methanol (650 mg, 4.08 mmol, WuXi AppTec), *N,N-*diisopropylethylamine (1.5 g, 11.6 mmol), and a 4A molecular sieve (1.4 g) were added. The reaction mixture was stirred for reaction at 105 °C for 6 h, cooled to room temperature, then filtered, and concentrated under reduced pressure to give a diastereomeric mixture of crude title compounds **1q-1 and 1q-2** (1.68 g, yield: 99.8%). The product was directly used in the next step without purification.

MS m/z (ESI): 729.2[M+1].

### Step 16

### Diastereomeric mixture of tert-butyl (5aS,6S,9R)-2-chloro-1-fluoro-12-(((2R, 7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-14-carboxylate 1r-1 and tert-butyl (5aR,6R,9S)- 2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-14-carboxylate 1r-2

The diastereomeric mixture of crude products **1q-1** and **1q-2** (1.68 g, 2.3 mmol) was added to tetrabutylammonium fluoride (2.59 g, 11.51 mmol). The reaction mixture was stirred at room temperature for 16 h and concentrated under reduced pressure, and the residue was purified with eluent system B to give a diastereomeric mixture of title compounds **1r-1** and **1r-2** (1.0 g, yield: 75.0%).

MS m/z (ESI): 579.2[M+1].

### Step 17

### Diastereomeric (1:1) mixture of tert-butyl (5aS,6S,9R)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-14-carboxylate 1s-1 and tert-butyl (5aR,6R,9S)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-14-carboxylate 1s-2

The diastereomeric mixture of compounds **1r-1** and **1r-2** (300 mg, 518.1 µmol), 2-(8-ethyl-7-fluoro-3-(methoxymethoxy)naphthalen-1-yl)-4,4,5,5-tetramethyl-1,3,2-dioxaborolane (280 mg, 777.2 µmol, prepared using the method for intermediate 18 disclosed on page 104 of the specification in Patent Application "WOO2021/041671"), tetrakis(triphenylphosphine)palladium (120 mg, 103.8 µmol), and cesium carbonate (506 mg, 1.55 mmol) were dissolved in 6 mL of a mixed solution of 1,4-dioxane and water (V:V = 5:1). The reaction mixture was reacted at 100 °C for 6 h under a nitrogen atmosphere and concentrated under reduced pressure to give a diastereomeric (1:1) mixture of crude title compounds **1s-1 and 1s-2** (400 mg). The product was directly used in the next step without purification.

MS m/z (ESI): 777.2[M+1].

### Step 18

### 5-Ethyl-6-fluoro-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 1-p1 and 5-ethyl-6-fluoro-4-((5aR,6R,9S)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 1-p2

The diastereomeric mixture of crude compounds **1s-1 and 1s-2** (160 mg, 205.9 µmol) was dissolved in ethyl acetate (5 mL), and 1 mL of a 4 M solution of hydrochloric acid in dioxane was added. The reaction mixture was reacted at 0 °C for 2 h and concentrated under reduced pressure, and the residue was purified by high performance liquid chromatography (Waters-2545, chromatography column: SharpSil-T C18, 30 × 150 mm, 5 µm; mobile phase: aqueous phase (10 mmol/L ammonium bicarbonate) and acetonitrile, gradient ratio: acetonitrile 38%-45%, flow rate: 30 mL/min) to give a diastereomeric (1:1) mixture of title compounds **1-p1** and **1-p2** (10 mg, yield: 7.2%).

MS m/z (ESI): 633.2[M+1].

¹H NMR (500 MHz, CD₃OD): δ 7.67 (ddd, 1H), 7.32-7.21 (m, 2H), 7.11-7.01 (m, 1H), 5.38-5.35 (m, 2H), 5.11-5.03 (m, 1H), 4.64-4.59 (m, 1H), 4.52-4.46 (m, 1H), 4.34-4.29 (m, 1H), 4.25 (dd, 1H), 4.18-4.12 (m, 1H), 3.74 (br, 1H), 3.65 (br, 1H), 3.26-3.23 (m, 3H), 3.05-3.01 (m, 1H), 2.61-1.81 (m, 12H), 0.94-0.82 (m, 3H).

The diastereomeric mixture of compounds **1-p1** and **1-p2** was subjected to chiral resolution (Shimadzu LC-20AP, chromatography column: DAICEL CHIRALPAK^{®}IC, 25 × 250 mm, 10 µm; mobile phase A: n-hexane, mobile phase B: ethanol (0.1% 7 M NH3 in MeOH)), gradient ratio: A:B: 40:60, flow rate: 30 mL/min) to give title compounds **1-p1** (26 mg, yield: 43.3%) and **1-p2** (26 mg, yield: 43.3%).

Single configuration compound (shorter retention time) **1-p2:** (26 mg, yield: 43.3%).

MS m/z (ESI): 633.2[M+1].

Chiral HPLC analysis: retention time: 7.89 min; purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.67 (ddd, 1H), 7.32-7.21 (m, 2H), 7.11-7.01 (m, 1H), 5.38-5.27 (m, 2H), 5.11-5.03 (m, 1H), 4.64-4.59 (m, 1H), 4.52-4.44 (m, 1H), 4.33 (d, 1H), 4.24 (dd, 1H), 4.18-4.12 (m, 1H), 3.75 (br, 1H), 3.66 (br, 1H), 3.27-3.18 (m, 3H), 3.05-3.03 (m, 1H), 2.60-1.81 (m, 12H), 0.93-0.82 (m, 3H).

Single configuration compound (longer retention time) **1-p1:** (26 mg, yield: 43.3%).

MS m/z (ESI): 633.2[M+1].

Chiral HPLC analysis: retention time: 13.8 min; purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.67 (ddd, 1H), 7.32-7.21 (m, 2H), 7.11-7.01 (m, 1H), 5.38-5.35 (m, 2H), 5.11-5.03 (m, 1H), 4.64-4.59 (m, 1H), 4.52-4.46 (m, 1H), 4.34-4.29 (m, 1H), 4.25 (dd, 1H), 4.18-4.12 (m, 1H), 3.74 (br, 1H), 3.65 (br, 1H), 3.26-3.23 (m, 3H), 3.05-3.01 (m, 1H), 2.61-1.81 (m, 12H), 0.94-0.82 m, 3H).

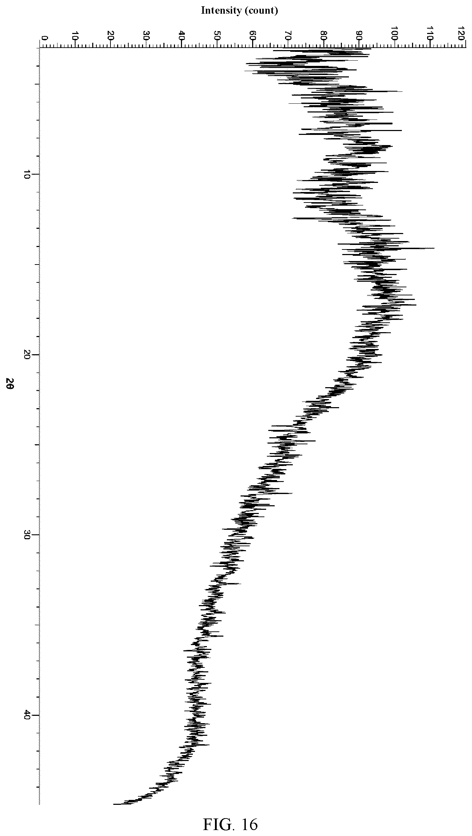

Compound **1-p1,** namely the compound represented by formula (I) in the present disclosure, was an amorphous form according to an X-ray powder diffraction analysis, and the X-ray powder diffraction pattern of the amorphous form is shown in FIG. 16.

### Biological Evaluations

### Test Example 1: Biological Evaluation of Inhibition of ERK Phosphorylation in AGS Cells

### (HTRF Method)

### I. Objective

In the experiment, the inhibitory effect of the compound on the KRAS target was evaluated by assessing the inhibitory effect of compound **1-p1** on ERK phosphorylation in cells and based on the IC₅₀ value.

### II. Experimental method

AGS cells (Nanjing Cobioer, CBP60476) were cultured in an RPMI1640 (Hyclone, SH30809.01) complete culture medium containing 10% fetal bovine serum. On the first day of the experiment, the AGS cells were seeded into a 96-well plate with a complete culture medium at a density of 40000 cells/well, with each well containing 190 µL of a cell suspension. The plate was incubated overnight in a 5% CO₂ cell incubator at 37 °C.

The next day, the test compound, serially diluted in a complete culture medium, was added at 10 µL/well. The final concentrations of the compound were 9 concentration points obtained by 5-fold serial dilution from 10 µM. A blank control containing 0.5% DMSO was set. The plate was incubated in a 5% CO₂ cell incubator at 37 °C for 1 h. After the incubation, the 96-well cell culture plate was taken out, and the culture medium was removed using a pipette. The plate was washed once by adding 200 µL of PBS (Shanghai BasalMedia Technologies Co., Ltd., B320) to each well. The PBS was removed using a pipette, and 50 µL of a lysis buffer (Cisbio, 64KL1FDF) containing a blocking reagent (Cisbio, 64KB1AAC) was added to each well. The plate was shaken on a shaker at room temperature for 40 min for lysis. After lysis, the lysate was pipetted for good uniformity. 16 µL of the lysate in each well was transferred to two HTRF 96-well assay plates (Cisbio, 66PL96100), and then 4 µL of a premixed phosphorylated ERK1/2 antibody solution (Cisbio, 64AERPEG) or 4 µL of a premixed total ERK1/2 antibody solution (Cisbio, 64NRKPEG) was added to these two plates. The microplates were sealed with a plate sealer film, centrifuged for 1 min in a microplate centrifuge, and incubated overnight at room temperature in the dark.

On the third day, the fluorescence values at an excitation wavelength of 337 nm and emission wavelengths of 665 nm and 620 nm were obtained using an ENVISION multi-mode microplate reader (PerkinElmer, ENVISION).

### III. Data analysis and results

The IC₅₀ value for the inhibitory activity of the compound was calculated using Graphpad Prism software based on the compound concentration and the phosphorylated ERK/total ERK ratio. The results show that the IC₅₀ of compound **1-p1** was 0.4 nM, and the compound had a relatively good inhibitory effect on ERK phosphorylation in AGS cells.

### Test Example 2: Biological Evaluation of Inhibition of 3D Proliferation of GP2d and AGS Cells

### I. Objective

The inhibitory effect of compound **1-p1** on the KRAS target was evaluated by assessing the inhibitory effect of compound **1-p1** on the 3D proliferation of GP2d and AGS cells.

### II. Experimental method

GP2d cells (Nanjing Cobioer, CBP60010) were cultured in a complete culture medium, namely a DMEM/high glucose culture medium (Hyclone, SH30243.01) containing 10% fetal bovine serum (Corning, 35-076-CV). On the first day of the experiment, the GP2d cells were seeded into a 96-well low-adsorption plate (Corning, CLS7007-24EA) with a complete culture medium at a density of 1000 cells/well, with each well containing 90 µL of a cell suspension. The plate was centrifuged at 2000 rpm for 5 min at room temperature and then incubated overnight in a 5% CO₂ cell incubator at 37 °C.

AGS cells (Nanjing Cobioer, CBP60476) were cultured in a complete culture medium, namely an RPMI1640 culture medium (Hyclone, SH30809.01) containing 10% fetal bovine serum (Corning, 35-076-CV). On the first day of the experiment, the AGS cells were seeded into a 96-well low-adsorption plate (Corning, CLS7007-24EA) with a complete culture medium at a density of 1000 cells/well, with each well containing 90 µL of a cell suspension. The plate was centrifuged at 2000 rpm for 5 min at room temperature and then incubated overnight in a 5% CO₂ cell incubator at 37 °C.

The next day, the test compound, serially diluted in a complete culture medium, was added at 10 µL/well. The final concentrations of the compound for GP2d cells were 9 concentration points obtained by 3-fold serial dilution from 1 µM, and the final concentrations of the compound for AGS cells were 9 concentration points obtained by 3-fold serial dilution from 10 µM. A blank control containing 0.5% DMSO was set for both types of cells. The plates were incubated in a 5% CO₂ cell incubator at 37 °C for 120 h. On the seventh day, the 96-well cell culture plates were taken out, and 50 µL of the CellTiter-Glo^{®} 3D reagent (Promega, G9682) was added to each well. The plates were shaken at room temperature for 25 min. The contents in the wells were pipetted for good uniformity, and 50 µL of the mixture was transferred into a white impermeable 96-well plate (PE, 6005290). The luminescence signal values were obtained using a multi-mode microplate reader (PerkinElmer, ENVISION).

### III. Data analysis and results

The IC₅₀ values for the inhibitory activity of the compound were calculated using Graphpad Prism software. The results show that the IC₅₀ in AGS cells was 5.8 nM, the IC₅₀ in GP2d cells was 0.9 nM, and compound **1-p1** had a relatively good inhibitory effect on the 3D proliferation of AGS and GP2d cells.

### Test Example 3: Determination of Affinity of Compound for KRAS Protein Subtype G12D or WT by SPR Method

Biotinylated Avi-KRAS-WT or Avi-KRAS-G12D was diluted to 20 µg/mL with a 1× HBS-P+ (Cat. # BR1006-71) buffer containing 100 mM MgCl₂ and then allowed to flow through channel 2 of an SA (Cat. # BR1005-31) biosensor chip for 420 s to obtain a coupling level of approximately 5000-7000 RU. The compound sample was injected for 120 s, followed by 720 s of dissociation. The testing was performed using a single-cycle kinetic mode. Reaction signals were detected in real time using a Biacore 8K instrument to obtain association and dissociation curves. After the testing was finished, data analysis was performed using Biacore 8K evaluation software. Data fitting was performed using a 1:1 model, and affinity data were obtained. The results show that the KD value of compound **1-p1** for KRAS G12D was 0.03E-09 mol/L, the KD value for WT was 1.54E-09 mol/L, and compound **1-p1** had relatively good affinity for KRAS protein subtype G12D or WT.

### Example 3: Preparation of Crystal Form A of Compound Represented by Formula (I)

8 mg of the compound represented by formula (I) was weighed and dissolved in 0.04 mL of methanol, and the mixture was cooled to 5 °C, stirred for crystallization, and centrifuged. The solid was dried *in vacuo* to give a product. The product was defined as the crystal form A of the compound represented by formula (I) by X-ray powder diffraction, with the XRPD pattern shown in FIG. 1 and the characteristic peak positions shown in Table 1. Its DSC thermogram showed endothermic peaks at 74.81 °C, 140.13 °C, and 196.78 °C.

**Table 1**

| Peak No. | 2θ [°] | d [Å] | Relative intensity% |
|---|---|---|---|
| 1 | 6.557 | 13.46957 | 22.1 |
| 2 | 9.207 | 9.59761 | 100.0 |
| 3 | 10.066 | 8.78057 | 24.8 |
| 4 | 10.681 | 8.27653 | 16.6 |
| 5 | 13.108 | 6.74873 | 37.9 |
| 6 | 14.533 | 6.08998 | 51.3 |
| 7 | 15.078 | 5.87118 | 24.1 |
| 8 | 16.639 | 5.32376 | 39.5 |
| 9 | 17.723 | 5.00038 | 47.3 |
| 10 | 19.566 | 4.53340 | 12.1 |
| 11 | 20.903 | 4.24631 | 24.9 |
| 12 | 21.547 | 4.12081 | 61.8 |
| 13 | 22.231 | 3.99565 | 38.7 |
| 14 | 24.030 | 3.70032 | 11.5 |
| 15 | 24.512 | 3.62875 | 8.1 |
| 16 | 24.943 | 3.56691 | 11.7 |
| 17 | 25.891 | 3.43851 | 11.0 |
| 18 | 27.241 | 3.27106 | 7.6 |
| 19 | 28.613 | 3.11722 | 10.3 |
| 20 | 30.186 | 2.95829 | 9.2 |

### Example 4: Preparation of Crystal Form A of Compound Represented by Formula (I)

8 mg of the compound represented by formula (I) was weighed and dissolved in 0.04 mL of 10% water/methanol (v/v), and the mixture was cooled to 5 °C, stirred for crystallization, and centrifuged. The solid was dried *in vacuo* to give a product. The product was identified as the crystal form A of the compound represented by formula (I) by X-ray powder diffraction.

### Example 5: Preparation of Crystal Form B of Compound Represented by Formula (I)

8 mg of the compound represented by formula (I) was added to 0.04 mL of ethanol, and the mixture was stirred for dissolution at room temperature, cooled to 5 °C, stirred for precipitation, and centrifuged. The solid was dried *in vacuo* to give a product. The product was defined as the crystal form B of the compound represented by formula (I) by X-ray powder diffraction, with the XRPD pattern shown in FIG. 2 and the characteristic peak positions shown in Table 2. Its DSC thermogram showed an endothermic peak at 226.81 °C.

**Table 2**

| Peak No. | 2θ [°] | d [Å] | Relative intensity% |
|---|---|---|---|
| 1 | 7.300 | 12.10023 | 14.1 |
| 2 | 8.790 | 10.05180 | 31.0 |
| 3 | 10.059 | 8.78668 | 8.2 |
| 4 | 10.595 | 8.34297 | 14.2 |
| 5 | 12.699 | 6.96537 | 67.9 |
| 6 | 14.565 | 6.07686 | 60.3 |
| 7 | 14.866 | 5.95443 | 40.1 |
| 8 | 16.200 | 5.46707 | 27.4 |
| 9 | 16.630 | 5.32655 | 13.9 |
| 10 | 17.303 | 5.12090 | 9.7 |
| 11 | 17.951 | 4.93753 | 89.7 |
| 12 | 19.220 | 4.61430 | 7.2 |
| 13 | 19.968 | 4.44311 | 29.7 |
| 14 | 21.415 | 4.14595 | 100.0 |
| 15 | 22.152 | 4.00960 | 23.7 |
| 16 | 24.430 | 3.64075 | 21.6 |
| 17 | 24.959 | 3.56474 | 12.9 |
| 18 | 25.733 | 3.45921 | 5.9 |
| 19 | 28.655 | 3.11273 | 10.2 |
| 20 | 29.379 | 3.03766 | 11.8 |

### Example 6: Preparation of Crystal Form B of Compound Represented by Formula (I)

8 mg of the compound represented by formula (I) was added to 0.8 mL of ethyl acetate/n-heptane (v/v = 1:1), and the mixture was stirred at room temperature for 2 days and centrifuged. The solid was dried *in vacuo* to give a product. The product was identified as the crystal form B of the compound represented by formula (I) by X-ray powder diffraction.

### Example 7: Preparation of Crystal Form C of Compound Represented by Formula (I)

8 mg of the compound represented by formula (I) was dissolved in 0.8 mL of acetonitrile at 50 °C, and the mixture was cooled to 5 °C, stirred for crystallization, and centrifuged. The solid was dried *in vacuo* to give a product. The product was defined as the crystal form C of the compound represented by formula (I) by X-ray powder diffraction, with the XRPD pattern shown in FIG. 3 and the characteristic peak positions shown in Table 3. Its DSC thermogram showed an endothermic peak at 198.44 °C.

**Table 3**

| Peak No. | 2θ [°] | d [Å] | Relative intensity% |
|---|---|---|---|
| 1 | 5.685 | 15.53374 | 45.3 |
| 2 | 6.432 | 13.73166 | 18.5 |
| 3 | 6.946 | 12.71585 | 16.1 |
| 4 | 7.579 | 11.65583 | 100.0 |
| 5 | 11.099 | 7.96534 | 51.7 |
| 6 | 11.433 | 7.73319 | 65.0 |
| 7 | 12.676 | 6.97771 | 14.5 |
| 8 | 13.936 | 6.34969 | 31.9 |
| 9 | 15.922 | 5.56188 | 10.7 |
| 10 | 17.242 | 5.13894 | 14.9 |
| 11 | 17.909 | 4.94883 | 41.7 |
| 12 | 19.312 | 4.59246 | 41.1 |
| 13 | 23.936 | 3.71472 | 19.7 |
| 14 | 24.405 | 3.64437 | 14.7 |
| 15 | 25.703 | 3.46317 | 6.2 |
| 16 | 26.375 | 3.37650 | 9.6 |

### Example 9: Preparation of Crystal Form D of Compound Represented by Formula (I)

8 mg of the compound represented by formula (I) was added to 0.05 mL of ethanol, and the mixture was stirred for dissolution at room temperature. 0.3 mL of isopropyl acetate was added, and the resulting mixture was stirred for crystallization and centrifuged. The solid was dried *in vacuo* to give a product. The product was defined as the crystal form D of the compound represented by formula (I) by X-ray powder diffraction, with the XRPD pattern shown in FIG. 4 and the characteristic peak positions shown in Table 4. Its DSC thermogram showed an endothermic peak at 178.74 °C.

**Table 4**

| Peak No. | 2θ [°] | d [Å] | Relative intensity% |
|---|---|---|---|
| 1 | 5.824 | 15.16194 | 41.5 |
| 2 | 6.708 | 13.16732 | 100.0 |
| 3 | 8.207 | 10.76514 | 16.7 |

### Example 10: Preparation of Crystal Form E of Compound Represented by Formula (I)

8 mg of the compound represented by formula (I) was dissolved in 0.6 mL of ethyl acetate, and the mixture was volatilized for crystallization to give a product. The product was defined as the crystal form E of the compound represented by formula (I) by X-ray powder diffraction, with the XRPD pattern shown in FIG. 5 and the characteristic peak positions shown in Table 5. Its DSC thermogram showed endothermic peaks at 44.15 °C, 167.45 °C, and 189.43 °C.

**Table 5**

| Peak No. | 2θ [°] | d [Å] | Relative intensity% |
|---|---|---|---|
| 1 | 6.745 | 13.09470 | 19.5 |
| 2 | 7.864 | 11.23310 | 100.0 |
| 3 | 9.422 | 9.37903 | 24.8 |
| 4 | 11.777 | 7.50816 | 7.1 |
| 5 | 12.336 | 7.16947 | 28.1 |
| 6 | 13.546 | 6.53141 | 8.2 |
| 7 | 13.992 | 6.32443 | 12.1 |
| 8 | 16.155 | 5.48217 | 25.6 |
| 9 | 17.521 | 5.05759 | 8.9 |
| 10 | 18.133 | 4.88819 | 12.4 |
| 11 | 20.341 | 4.36237 | 17.0 |
| 12 | 21.534 | 4.12333 | 6.8 |
| 13 | 23.617 | 3.76411 | 3.7 |
| 14 | 24.857 | 3.57910 | 2.7 |

### Example 11: Preparation of Crystal Form F of Compound Represented by Formula (I)

8 mg of the compound represented by formula (I) was added to 0.8 mL of methyl tert-butyl ether, and the mixture was stirred at room temperature for 2 days and centrifuged. The solid was dried *in vacuo* to give a product. The product was defined as the crystal form F of the compound represented by formula (I) by X-ray powder diffraction, with the XRPD pattern shown in FIG. 6 and the characteristic peak positions shown in Table 6. Its DSC thermogram showed endothermic peaks at 198.57 °C and 208.23 °C.

**Table 6**

| Peak No. | 2θ [°] | d [Å] | Relative intensity% |
|---|---|---|---|
| 1 | 5.664 | 15.59167 | 59.9 |
| 2 | 7.766 | 11.37533 | 100.0 |
| 3 | 10.432 | 8.47309 | 8.9 |
| 4 | 11.323 | 7.80822 | 79.8 |
| 5 | 12.409 | 7.12738 | 15.8 |
| 6 | 13.657 | 6.47885 | 48.6 |
| 7 | 15.553 | 5.69271 | 3.5 |
| 8 | 15.990 | 5.53815 | 15.2 |
| 9 | 17.099 | 5.18143 | 15.0 |
| 10 | 18.002 | 4.92358 | 25.9 |
| 11 | 18.978 | 4.67247 | 48.0 |
| 12 | 19.670 | 4.50974 | 15.8 |
| 13 | 23.307 | 3.81351 | 7.2 |
| 14 | 24.200 | 3.67475 | 13.7 |
| 15 | 25.019 | 3.55625 | 14.6 |
| 16 | 25.775 | 3.45367 | 9.9 |
| 17 | 26.877 | 3.31455 | 6.0 |

### Example 12: Preparation of Crystal Form G of Compound Represented by Formula (I)

8 mg of the compound represented by formula (I) was added to 0.8 mL of dichloromethane, and the mixture was stirred at room temperature for 2 days and centrifuged. The solid was dried *in vacuo* to give a product. The product was defined as the crystal form G of the compound represented by formula (I) by X-ray powder diffraction, with the XRPD pattern shown in FIG. 7 and the characteristic peak positions shown in Table 7. Its DSC thermogram showed an endothermic peak at 177.46 °C.

**Table 7**

| Peak No. | 2θ [°] | d [Å] | Relative intensity% |
|---|---|---|---|
| 1 | 7.662 | 11.52941 | 100.0 |
| 2 | 10.917 | 8.09802 | 5.8 |
| 3 | 12.832 | 6.89335 | 6.5 |
| 4 | 15.446 | 5.73217 | 24.5 |
| 5 | 20.520 | 4.32476 | 3.7 |
| 6 | 21.965 | 4.04343 | 4.7 |

### Example 13: Preparation of Crystal Form H of Compound Represented by Formula (I)

The crystal form E (Example 10) of the compound represented by formula (I) was heated to 180 °C to give a product. The product was defined as the crystal form H of the compound represented by formula (I) by X-ray powder diffraction, with the XRPD pattern shown in FIG. 8 and the characteristic peak positions shown in Table 8. Its DSC thermogram showed an endothermic peak at 193.39 °C.

**Table 8**

| Peak No. | 2θ [°] | d [Å] | Relative intensity% |
|---|---|---|---|
| 1 | 7.468 | 11.82736 | 8.5 |
| 2 | 8.594 | 10.28054 | 100.0 |
| 3 | 9.387 | 9.41440 | 7.3 |
| 4 | 12.307 | 7.18587 | 4.9 |
| 5 | 13.986 | 6.32701 | 11.4 |
| 6 | 15.889 | 5.57327 | 10.2 |
| 7 | 17.367 | 5.10223 | 8.1 |
| 8 | 18.391 | 4.82038 | 5.8 |
| 9 | 19.434 | 4.56397 | 16.0 |
| 10 | 20.490 | 4.33088 | 4.6 |
| 11 | 21.582 | 4.11416 | 3.2 |
| 12 | 22.913 | 3.87813 | 6.5 |
| 13 | 24.786 | 3.58925 | 3.8 |
| 14 | 26.557 | 3.35372 | 5.0 |
| 15 | 27.164 | 3.28018 | 4.5 |

### Example 13: Preparation of Crystal Form I of Compound Represented by Formula (I)

30 mg of the compound represented by formula (I) was dissolved in 0.3 mL of ethanol, and the mixture was stirred for precipitation at room temperature and centrifuged. The solid was dried *in vacuo* to give a product. The product was defined as the crystal form I of the compound represented by formula (I) by X-ray powder diffraction, with the XRPD pattern shown in FIG. 9 and the characteristic peak positions shown in Table 9. Its DSC thermogram showed an endothermic peak at 251.58 °C.

**Table 9**

| Peak No. | 2θ [°] | d [Å] | Relative intensity% |
|---|---|---|---|
| 1 | 5.643 | 15.64851 | 7.2 |
| 2 | 6.928 | 12.74965 | 100.0 |
| 3 | 8.317 | 10.62253 | 6.1 |
| 4 | 9.649 | 9.15885 | 4.3 |
| 5 | 11.353 | 7.78754 | 8.4 |
| 6 | 13.906 | 6.36329 | 7.5 |
| 7 | 15.349 | 5.76805 | 18.6 |
| 8 | 16.551 | 5.35172 | 16.4 |
| 9 | 18.392 | 4.81993 | 5.7 |
| 10 | 20.059 | 4.42300 | 17.1 |
| 11 | 20.585 | 4.31117 | 6.7 |
| 12 | 21.068 | 4.21354 | 2.7 |
| 13 | 22.130 | 4.01368 | 3.6 |
| 14 | 23.127 | 3.84280 | 4.0 |
| 15 | 24.454 | 3.63722 | 6.9 |
| 16 | 25.976 | 3.42737 | 3.2 |
| 17 | 33.014 | 2.71103 | 2.7 |

### Example 15: Preparation of Crystal Form I of Compound Represented by Formula (I)

8 mg of the compound represented by formula (I) was added to 0.8 mL of acetonitrile, and the mixture was stirred at room temperature for 2 days and filtered. The solid was dried *in vacuo* to give a product. The product was identified as the crystal form I of the compound represented by formula (I) by X-ray powder diffraction.

### Example 16: Preparation of Crystal Form I of Compound Represented by Formula (I)

8 mg of the compound represented by formula (I) was dissolved in 0.04 mL of ethyl acetate, and the mixture was stirred at room temperature overnight and filtered. The solid was dried *in vacuo* to give a product. The product was identified as the crystal form I of the compound represented by formula (I) by X-ray powder diffraction.

### Example 17: Preparation of Crystal Form J of Compound Represented by Formula (I)

60 mg of the compound represented by formula (I) was added to 0.5 mL of dichloromethane, and the mixture was stirred at room temperature overnight and filtered. The solid was dried *in vacuo* to give a product. The product was defined as the crystal form J of the compound represented by formula (I) by X-ray powder diffraction, with the XRPD pattern shown in FIG. 10 and the characteristic peak positions shown in Table 10. Its DSC thermogram showed an endothermic peak at 208.11 °C.

**Table 10**

| Peak No. | 2θ [°] | d [Å] | Relative intensity% |
|---|---|---|---|
| 1 | 7.217 | 12.23881 | 30.4 |
| 2 | 7.675 | 11.50971 | 25.0 |
| 3 | 8.462 | 10.44068 | 100.0 |
| 4 | 9.844 | 8.97772 | 24.7 |
| 5 | 10.584 | 8.35194 | 28.6 |
| 6 | 12.153 | 7.27687 | 7.1 |
| 7 | 13.527 | 6.54070 | 12.8 |
| 8 | 14.903 | 5.93978 | 27.7 |
| 9 | 15.745 | 5.62402 | 7.6 |
| 10 | 17.074 | 5.18902 | 19.1 |
| 11 | 17.946 | 4.93880 | 6.5 |
| 12 | 19.126 | 4.63656 | 15.1 |
| 13 | 19.954 | 4.44605 | 4.9 |
| 14 | 20.483 | 4.33251 | 19.4 |
| 15 | 21.615 | 4.10807 | 7.9 |
| 16 | 23.314 | 3.81235 | 8.1 |
| 17 | 24.280 | 3.66289 | 3.2 |
| 18 | 25.129 | 3.54094 | 8.1 |
| 19 | 25.747 | 3.45734 | 4.0 |
| 20 | 28.340 | 3.14662 | 6.5 |

### Example 18: Preparation of Crystal Form K of Compound Represented by Formula (I)

8 mg of the compound represented by formula (I) was dissolved in 0.04 mL of tetrahydrofuran, and the mixture was stirred for precipitation at room temperature and centrifuged. The solid was dried *in vacuo* to give a product. The product was defined as the crystal form K of the compound represented by formula (I) by X-ray powder diffraction, with the XRPD pattern shown in FIG. 11 and the characteristic peak positions shown in Table 11. Its DSC thermogram showed an endothermic peak at 208.48 °C.

**Table 11**

| Peak No. | 2θ [°] | d [Å] | Relative intensity% |
|---|---|---|---|
| 1 | 5.323 | 16.58824 | 3.1 |
| 2 | 6.717 | 13.14956 | 100.0 |
| 3 | 10.668 | 8.28596 | 1.7 |
| 4 | 12.314 | 7.18193 | 0.8 |
| 5 | 13.549 | 6.52985 | 5.9 |
| 6 | 14.805 | 5.97871 | 1.5 |
| 7 | 18.145 | 4.88509 | 0.9 |
| 8 | 20.556 | 4.31728 | 0.7 |
| 9 | 21.313 | 4.16564 | 0.8 |
| 10 | 27.340 | 3.25948 | 0.7 |

### Example 19: Preparation of Crystal Form L of Compound Represented by Formula (I)

60 mg of the compound represented by formula (I) was dissolved in 1 mL of ethyl acetate, and the mixture was stirred for precipitation at room temperature and filtered. The solid was dried *in vacuo* to give a product. The product was defined as the crystal form L of the compound represented by formula (I) by X-ray powder diffraction, with the XRPD pattern shown in FIG. 12 and the characteristic peak positions shown in Table 12. Its DSC thermogram showed endothermic peaks at 57.12 °C, 137.09 °C, and 193.02 °C.

**Table 12**

| Peak No. | 2θ [°] | d [Å] | Relative intensity% |
|---|---|---|---|
| 1 | 6.807 | 12.9751 | 30.8 |
| 2 | 8.426 | 10.4856 | 100.0 |
| 3 | 9.459 | 9.3421 | 55.3 |
| 4 | 12.303 | 7.1887 | 31.5 |
| 5 | 14.012 | 6.3153 | 41.5 |
| 6 | 16.826 | 5.2648 | 24.1 |
| 7 | 18.282 | 4.8487 | 32.7 |
| 8 | 20.260 | 4.3797 | 17.9 |
| 9 | 21.709 | 4.0905 | 17.9 |
| 10 | 22.743 | 3.9067 | 2.9 |
| 11 | 24.764 | 3.5923 | 3.5 |

### Example 20: Preparation of Crystal Form M of Compound Represented by Formula (I)

60 mg of the compound represented by formula (I) was dispersed in 5 mL of isopropyl acetate, and the mixture was stirred at room temperature for 5 days and filtered. The solid was dried *in vacuo* to give a product. The product was defined as the crystal form M of the compound represented by formula (I) by X-ray powder diffraction, with the XRPD pattern shown in FIG. 13 and the characteristic peak positions shown in Table 13. Its DSC thermogram showed endothermic peaks at 45.80 °C, 130.75 °C, and 193.53 °C.

**Table 13**

| Peak No. | 2θ [°] | d [Å] | Relative intensity% |
|---|---|---|---|
| 1 | 6.737 | 13.1108 | 57.5 |
| 2 | 7.844 | 11.2614 | 100.0 |
| 3 | 9.520 | 9.2824 | 26.3 |
| 4 | 11.894 | 7.4350 | 18.2 |
| 5 | 12.343 | 7.1653 | 38.2 |
| 6 | 13.019 | 6.7947 | 4.2 |
| 7 | 13.630 | 6.4915 | 9.9 |
| 8 | 14.287 | 6.1946 | 14.3 |
| 9 | 14.917 | 5.9342 | 9.5 |
| 10 | 15.754 | 5.6205 | 15.2 |
| 11 | 16.813 | 5.2689 | 22.2 |
| 12 | 17.700 | 5.0069 | 15.7 |
| 13 | 18.276 | 4.8504 | 32.2 |
| 14 | 19.123 | 4.6374 | 5.4 |
| 15 | 20.353 | 4.3599 | 17.7 |
| 16 | 21.698 | 4.0925 | 18.4 |
| 17 | 22.407 | 3.9647 | 1.6 |
| 18 | 23.935 | 3.7148 | 15.1 |
| 19 | 24.929 | 3.5689 | 7.2 |
| 20 | 26.678 | 3.3388 | 8.2 |
| 21 | 27.374 | 3.2555 | 1.6 |
| 22 | 27.879 | 3.1976 | 2.3 |

### Example 21: Preparation of Crystal Form N of Compound Represented by Formula (I)

60 mg of the compound represented by formula (I) was dissolved in 1 mL of acetone, and the mixture was stirred for precipitation at room temperature and filtered. The solid was dried *in vacuo* to give a product. The product was defined as the crystal form N of the compound represented by formula (I) by X-ray powder diffraction, with the XRPD pattern shown in FIG. 14 and the characteristic peak positions shown in Table 14. Its DSC thermogram showed endothermic peaks at 76.42 °C and 143.82 °C.

**Table 14**

| Peak No. | 2θ [°] | d [Å] | Relative intensity% |
|---|---|---|---|
| 1 | 6.645 | 13.2915 | 15.6 |
| 2 | 7.735 | 11.4211 | 10.9 |
| 3 | 8.226 | 10.7402 | 100.0 |
| 4 | 9.052 | 9.7612 | 15.8 |
| 5 | 10.083 | 8.7654 | 42.1 |
| 6 | 11.277 | 7.8403 | 21.9 |
| 7 | 11.841 | 7.4681 | 11.8 |
| 8 | 12.647 | 6.9938 | 7.5 |
| 9 | 13.973 | 6.3329 | 30.9 |
| 10 | 15.393 | 5.7516 | 13.3 |
| 11 | 17.768 | 4.9880 | 37.8 |
| 12 | 19.015 | 4.6636 | 20.1 |
| 13 | 19.887 | 4.4610 | 21.3 |
| 14 | 21.743 | 4.0842 | 21.9 |
| 15 | 23.080 | 3.8505 | 4.6 |
| 16 | 24.048 | 3.6977 | 12.5 |
| 17 | 25.564 | 3.4817 | 5.4 |

### Example 22: Preparation of Crystal Form O of Compound Represented by Formula (I)

8 mg of the compound represented by formula (I) was dissolved in 0.12 mL of isopropanol, and the mixture was stirred for precipitation at room temperature and centrifuged. The solid was dried *in vacuo* to give a product. The product was defined as the crystal form O of the compound represented by formula (I) by X-ray powder diffraction, with the XRPD pattern shown in FIG. 15 and the characteristic peak positions shown in Table 15. Its DSC thermogram showed endothermic peaks at 120.14 °C and 177.32 °C.

**Table 15**

| Peak No. | 2θ [°] | d [Å] | Relative intensity% |
|---|---|---|---|
| 1 | 6.959 | 12.69178 | 100.0 |
| 2 | 7.489 | 11.79457 | 7.3 |
| 3 | 8.920 | 9.90624 | 21.0 |
| 4 | 9.668 | 9.14096 | 6.1 |
| 5 | 10.479 | 8.43516 | 15.2 |
| 6 | 12.031 | 7.35038 | 7.8 |
| 7 | 12.802 | 6.90957 | 10.3 |
| 8 | 13.182 | 6.71085 | 12.3 |
| 9 | 14.039 | 6.30310 | 10.2 |
| 10 | 14.449 | 6.12533 | 5.0 |
| 11 | 15.080 | 5.87028 | 6.3 |
| 12 | 17.488 | 5.06701 | 9.9 |
| 13 | 18.137 | 4.88724 | 10.4 |
| 14 | 18.777 | 4.72193 | 17.5 |
| 15 | 19.642 | 4.51594 | 11.5 |
| 16 | 20.588 | 4.31049 | 4.7 |
| 17 | 21.175 | 4.19235 | 25.2 |
| 18 | 22.342 | 3.97602 | 2.0 |
| 19 | 24.201 | 3.67460 | 6.7 |
| 20 | 24.982 | 3.56147 | 3.1 |
| 21 | 25.927 | 3.43377 | 4.3 |
| 22 | 27.706 | 3.21723 | 1.8 |
| 23 | 28.289 | 3.15217 | 2.3 |

### Example 23: Crystal Form Stability Study

Samples of the crystal forms A, B, C, I, J, and K of the compound represented by formula (I) were taken, spread in open containers, and left to stand under conditions of light exposure (4500 Lux), high temperatures (40 °C and 60 °C), and high humidities (RH 75% and RH 92.5%) for 30 days to investigate their stability, and the results are shown in Table 16.

**Table 16**

| Condition | Time (days) | Crystal form A | | |
|---|---|---|---|---|
| | | Color and state | Purity% | Crystal form |
| Initial | 0 | Off-white solid | 99.3 | A |
| | 5 | Off-white solid | 99.2 | Did not change |
| 40 °C | 10 | Off-white solid | 99.2 | Did not change |
| | 30 | Off-white solid | 99.1 | Did not change |
| | 5 | Off-white solid | 99.1 | Did not change |
| 60 °C | 10 | Off-white solid | 99.0 | Did not change |
| | 30 | Off-white solid | 98.5 | Did not change |
| | 5 | Off-white solid | 99.3 | Did not change |
| 75% RH | 10 | Off-white solid | 99.3 | Did not change |
| | 30 | Off-white solid | 99.3 | Did not change |
| | 5 | Off-white solid | 99.3 | Did not change |
| 92.5% RH | 10 | Off-white solid | 99.4 | Did not change |
| | 30 | Off-white solid | 99.3 | Did not change |
| | 5 | Off-white solid | 98.7 | Did not change |
| 4500 Lux | 10 | Off-white solid | 98.5 | Did not change |
| | 30 | Pale yellow solid | 95.8 | Did not change |

| Condition | Time (days) | Crystal form B | | |
|---|---|---|---|---|
| | | Color and state | Purity% | Crystal form |
| Initial | 0 | Off-white solid | 99.4 | B |
| | 5 | Off-white solid | 99.3 | Did not change |
| 40 °C | 10 | Off-white solid | 99.3 | Did not change |
| | 30 | Off-white solid | 99.2 | Did not change |
| | 5 | Off-white solid | 99.3 | Did not change |
| 60 °C | 10 | Off-white solid | 99.2 | Did not change |
| | 30 | Off-white solid | 99.1 | Did not change |
| | 5 | Off-white solid | 99.3 | Did not change |
| 75% RH | 10 | Off-white solid | 99.4 | Did not change |
| | 30 | Off-white solid | 99.3 | Did not change |
| | 5 | Off-white solid | 99.4 | Did not change |
| 92.5% RH | 10 | Off-white solid | 99.4 | Did not change |
| | 30 | Off-white solid | 99.4 | Did not change |
| | 5 | Off-white solid | 99.0 | Did not change |
| 4500 Lux | 10 | Off-white solid | 99.1 | Did not change |
| | 30 | Pale yellow solid | 97.6 | Did not change |

| Condition | Time (days) | Crystal form C | | |
|---|---|---|---|---|
| | | Color and state | Purity% | Crystal form |
| Initial | 0 | Off-white solid | 99.7 | C |
| | 5 | Off-white solid | 99.3 | Did not change |
| 40 °C | 10 | Off-white solid | 99.3 | Did not change |
| | 30 | Off-white solid | 98.5 | Did not change |
| | 5 | Off-white solid | 99.0 | Did not change |
| 60 °C | 10 | Off-white solid | 99.0 | Did not change |
| | 30 | Off-white solid | 97.9 | Did not change |
| | 5 | Off-white solid | 99.6 | Did not change |
| 75% RH | 10 | Off-white solid | 99.6 | Did not change |
| | 30 | Off-white solid | 99.5 | Did not change |
| | 5 | Off-white solid | 99.6 | Did not change |
| 92.5% RH | 10 | Off-white solid | 99.6 | Did not change |
| | 30 | Off-white solid | 99.4 | Did not change |
| | 5 | Off-white solid | 99.2 | Did not change |
| 4500 Lux | 10 | Off-white solid | 98.7 | Did not change |
| | 30 | Pale yellow solid | 97.9 | Did not change |

| Condition | Time (days) | Crystal form I | | |
|---|---|---|---|---|
| | | Color and state | Purity% | Crystal form |
| Initial | 0 | Off-white solid | 99.6 | Crystal form I |
| | 5 | Off-white solid | 99.6 | Did not change |
| 40 °C | 10 | Off-white solid | 99.4 | Did not change |
| | 30 | Off-white solid | 99.3 | Did not change |
| | 5 | Off-white solid | 99.6 | Did not change |
| 60 °C | 10 | Off-white solid | 99.4 | Did not change |
| | 30 | Off-white solid | 99.3 | Did not change |
| | 5 | Off-white solid | 99.6 | Did not change |
| 75% RH | 10 | Off-white solid | 99.6 | Did not change |
| | 30 | Off-white solid | 99.6 | Did not change |
| | 5 | Off-white solid | 99.5 | Did not change |
| 92.5% RH | 10 | Off-white solid | 99.5 | Did not change |
| | 30 | Off-white solid | 99.6 | Did not change |
| Light | 5 | Off-white solid | 99.6 | Did not change |
| exposure | 10 | Off-white solid | 99.5 | Did not change |
| | 30 | Off-white solid | 99.2 | Did not change |

| Condition | Time (days) | Crystal form J | | |
|---|---|---|---|---|
| | | Color and state | Purity% | Crystal form |
| Initial | 0 | Off-white solid | 99.6 | Crystal form J |
| | 5 | Off-white solid | 99.2 | Did not change |
| 40 °C | 10 | Off-white solid | 98.8 | Did not change |
| | 30 | Off-white solid | 97.9 | Did not change |
| | 5 | Off-white solid | 99.1 | Did not change |
| 60 °C | 10 | Off-white solid | 98.5 | Did not change |
| | 30 | Off-white solid | 97.4 | Did not change |
| | 5 | Off-white solid | 99.6 | Did not change |
| 75% RH | 10 | Off-white solid | 99.6 | Did not change |
| | 30 | Off-white solid | 99.5 | Did not change |
| | 5 | Off-white solid | 99.6 | Did not change |
| 92.5% RH | 10 | Off-white solid | 99.5 | Did not change |
| | 30 | Off-white solid | 99.5 | Did not change |
| Light | 5 | Off-white solid | 98.1 | Did not change |
| exposure | 10 | Pale yellow solid | 95.5 | Did not change |
| | 30 | Pale yellow solid | 93.0 | Did not change |

| Condition | Time (days) | Crystal form K | | |
|---|---|---|---|---|
| | | Color and state | Purity% | Crystal form |
| Initial | 0 | Off-white solid | 99.8 | Crystal form K |
| | 5 | Off-white solid | 99.2 | Did not change |
| 40 °C | 10 | Off-white solid | 98.5 | Did not change |
| | 30 | Off-white solid | 97.0 | Did not change |
| | 5 | Off-white solid | 99.1 | Did not change |
| 60 °C | 10 | Off-white solid | 98.5 | Did not change |
| | 30 | Off-white solid | 97.0 | Did not change |
| | 5 | Off-white solid | 99.8 | Did not change |
| 75% RH | 10 | Off-white solid | 99.7 | Did not change |
| | 30 | Off-white solid | 99.7 | Did not change |
| | 5 | Off-white solid | 99.8 | Did not change |
| 92.5% RH | 10 | Off-white solid | 99.7 | Did not change |
| | 30 | Off-white solid | 99.7 | Did not change |
| Light | 5 | Off-white solid | 99.3 | Did not change |
| exposure | 10 | Off-white solid | 98.9 | Did not change |
| | 30 | Off-white solid | 97.9 | Did not change |

Conclusion: The influencing factor experiments show that: the crystal forms A, B, C, I, J, and K had good physical and chemical properties after being left to stand under conditions of high temperatures and high humidities for 30 days.

### Example 24: Long-Term/Accelerated Stability

The crystal forms A, B, C, I, J, and K of the compound represented by formula (I) were left to stand under conditions of 25 °C/60% RH and 40 °C/75% RH to investigate their stability, and the results are shown in Table 17.

**Table 17**

| Sample | Standing conditions | Purity% | Purity% | Purity% | Purity% | Crystal form |
|---|---|---|---|---|---|---|
| | | Initial | 1 month | 3 months | 6 months | |
| Crystal form A | 25 °C/60% RH | 99.3 | 99.3 | 99.3 | 99.3 | A |
| | 40 °C/75% RH | 99.3 | 99.3 | 99.2 | 99.1 | A |

| Sample | Standing conditions | Purity% | Purity% | Purity% | Purity% | Crystal form |
|---|---|---|---|---|---|---|
| | | Initial | 1 month | 3 months | 6 months | |
| Crystal | 25 °C/60% | 99.4 | 99.4 | 99.3 | 99.3 | B |
| form B | RH | | | | | |
| | 40 °C/75% RH | 99.4 | 99.3 | 99.2 | 99.1 | B |

| Sample | Standing conditions | Purity% | Purity% | Purity% | Purity% | Crystal form |
|---|---|---|---|---|---|---|
| | | Initial | 1 month | 2 months | 3 months | |
| Crystal form C | 25 °C/60% RH | 99.7 | 99.7 | 99.6 | 99.6 | C |
| | 40 °C/75% RH | 99.7 | 99.5 | 99.4 | 99.2 | C |

| Sample | Standing conditions | Purity% | Purity% | Purity% | Purity% | Crystal form |
|---|---|---|---|---|---|---|
| | | Initial | 1 month | 3 months | 2 months | |
| Crystal form I | 25 °C/60% RH | 99.6 | 99.6 | 99.6 | 99.6 | I |
| | 40 °C/75% RH | 99.6 | 99.6 | 99.6 | 99.4 | I |

| Sample | Standing conditions | Purity% | Purity% | Purity% | Purity% | Crystal form |
|---|---|---|---|---|---|---|
| | | Initial | 1 month | 2 months | 3 months | |
| Crystal form J | 25 °C/60% RH | 99.6 | 99.5 | 99.6 | 99.6 | J |
| | 40 °C/75% RH | 99.6 | 99.4 | 99.3 | 99.2 | J |

| Sample | Standing conditions | Purity% | Purity% | Purity% | Purity% | Crystal form |
|---|---|---|---|---|---|---|
| | | Initial | 1 month | 2 months | 6 months | |
| Crystal form K | 25 °C/60% RH | 99.8 | 99.7 | 99.8 | 99.7 | K |
| | 40 °C/75% RH | 99.8 | 99.7 | 99.7 | 99.4 | K |

Conclusion: The long-term accelerated experiments show that: the crystal forms A, B, C, I, J, and K exhibited good physical and chemical stability after being left to stand under conditions of 25 °C/60% RH and 40 °C/75% RH for 3 or 6 months.

## Claims

1. A crystal form A of the compound represented by formula (I), wherein an X-ray powder diffraction pattern of the crystal form has characteristic peaks at 2θ angles of 9.2, 13.1, 14.5, 16.6, 17.7, and 21.5.

2. The crystal form A of the compound represented by formula (I) according to claim 1, wherein the X-ray powder diffraction pattern of the crystal form has characteristic peaks at 2θ angles of 6.6, 9.2, 10.1, 10.7, 13.1, 14.5, 15.1, 16.6, 17.7, 19.6, 20.9, 21.5, 22.2, 24.0, 24.5, 24.9, 25.9, 27.2, 28.6, and 30.2.

3. The crystal form A of the compound represented by formula (I) according to claim 1, wherein the X-ray powder diffraction pattern of the crystal form is shown in FIG. 1.

4. A crystal form B of the compound represented by formula (I), wherein an X-ray powder diffraction pattern of the crystal form has characteristic peaks at 2θ angles of 8.8, 12.7, 14.6, 14.9, 16.2, 18.0, and 21.4.

5. The crystal form B of the compound represented by formula (I) according to claim 4, wherein the X-ray powder diffraction pattern of the crystal form has characteristic peaks at 2θ angles of 7.3, 8.8, 10.1, 10.6, 12.7, 14.6, 14.9, 16.2, 16.6, 17.3, 18.0, 19.2, 20.0, 21.4, 22.2, 24.4, 25.0, 25.7, 28.7, and 29.4.

6. The crystal form B of the compound represented by formula (I) according to claim 4, wherein the X-ray powder diffraction pattern of the crystal form is shown in FIG. 2.

7. A crystal form C of the compound represented by formula (I), wherein an X-ray powder diffraction pattern of the crystal form has characteristic peaks at 2θ angles of 5.7, 7.6, 11.1, 11.4, 17.9, and 19.3.

8. The crystal form C of the compound represented by formula (I) according to claim 7, wherein the X-ray powder diffraction pattern of the crystal form has characteristic peaks at 2θ angles of 5.7, 6.4, 6.9, 7.6, 11.1, 11.4, 12.7, 13.9, 15.9, 17.2, 17.9, 19.3, 23.9, 24.4, 25.7, and 26.4.

9. The crystal form C of the compound represented by formula (I) according to claim 7, wherein the X-ray powder diffraction pattern of the crystal form is shown in FIG. 3.

10. A crystal form I of the compound represented by formula (I), wherein an X-ray powder diffraction pattern of the crystal form has characteristic peaks at 2θ angles of 6.9, 15.3, 16.6, and 20.1.

11. The crystal form I of the compound represented by formula (I) according to claim 10, wherein the X-ray powder diffraction pattern of the crystal form has characteristic peaks at 2θ angles of 5.6, 6.9, 8.3, 9.6, 11.4, 13.9, 15.3, 16.6, 18.4, 20.1, 20.6, 21.1, 22.1, 23.1, 24.5, 26.0, and 33.0.

12. The crystal form I of the compound represented by formula (I) according to claim 10, wherein the X-ray powder diffraction pattern of the crystal form is shown in FIG. 9.

13. A crystal form J of the compound represented by formula (I), wherein an X-ray powder diffraction pattern of the crystal form has characteristic peaks at 2θ angles of 7.2, 7.7, 8.5, 9.8, 10.6, and 14.9.

14. The crystal form J of the compound represented by formula (I) according to claim 13, wherein the X-ray powder diffraction pattern of the crystal form has characteristic peaks at 2θ angles of 7.2, 7.7, 8.5, 9.8, 10.6, 12.2, 13.5, 14.9, 15.7, 17.1, 17.9, 19.1, 20.0, 20.5, 21.6, 23.3, 24.3, 25.1, 25.7, and 28.3.

15. The crystal form J of the compound represented by formula (I) according to claim 13, wherein the X-ray powder diffraction pattern of the crystal form is shown in FIG. 10.

16. A crystal form K of the compound represented by formula (I), wherein an X-ray powder diffraction pattern of the crystal form has characteristic peaks at 2θ angles of 5.3, 6.7, 10.7, 12.3, 13.5, 14.8, 18.1, 20.6, 21.3, and 27.3.

17. The crystal form K of the compound represented by formula (I) according to claim 16, wherein the X-ray powder diffraction pattern of the crystal form is shown in FIG. 11.

18. The crystal form of the compound represented by formula (I) according to any one of claims 1-17, wherein the 2θ angles have a margin of error of ±0.2.

19. A method for preparing the crystal form A of the compound represented by formula (I) according to any one of claims 1-3 and 18, comprising: mixing the compound represented by formula (I) with a proper amount of a solvent, and cooling for crystallization, wherein the solvent is selected from the group consisting of one or more of methanol and water/methanol.

20. A method for preparing the crystal form B of the compound represented by formula (I) according to any one of claims 4-6 and 18, comprising: mixing the compound represented by formula (I) with a proper amount of a solvent, and cooling for crystallization, wherein the solvent is selected from the group consisting of one or more of ethanol, isopropanol, n-propanol, ethyl acetate/ethanol, and ethyl acetate/n-heptane.

21. A method for preparing the crystal form C of the compound represented by formula (I) according to any one of claims 7-9 and 18, comprising: mixing the compound represented by formula (I) with a proper amount of a solvent, and crystallizing, wherein the solvent is acetonitrile.

22. A method for preparing the crystal form I of the compound represented by formula (I) according to any one of claims 10-12 and 18, comprising: mixing the compound represented by formula (I) with a proper amount of a solvent, and stirring at room temperature for crystallization, wherein the solvent is selected from the group consisting of one or more of methanol, ethanol, n-propanol, water/methanol, water/ethanol, water/isopropanol, acetonitrile/methanol, acetone, water/acetone, 2-butanone, acetonitrile, ethyl acetate, isopropyl acetate, *n*-heptane, tetrahydrofuran/ethanol, ethyl acetate/ethanol, ethyl acetate/*n*-heptane, isopropyl ether, and methyl *tert*-butyl ether.

23. A method for preparing the crystal form J of the compound represented by formula (I) according to any one of claims 13-15 and 18, comprising: mixing the compound represented by formula (I) with a proper amount of a solvent, and crystallizing, wherein the solvent is dichloromethane.

24. A method for preparing the crystal form K of the compound represented by formula (I) according to any one of claims 16-18, comprising: mixing the compound represented by formula (I) with a proper amount of a solvent, and crystallizing, wherein the solvent is tetrahydrofuran.

25. A pharmaceutical composition, comprising the following components:
(a) the crystal form A of the compound represented by formula (I) according to any one of claims 1-3 and 18, the crystal form B of the compound represented by formula (I) according to any one of claims 4-6 and 18, the crystal form C of the compound represented by formula (I) according to any one of claims 7-9 and 18, the crystal form I of the compound represented by formula (I) according to any one of claims 10-12 and 18, the crystal form J of the compound represented by formula (I) according to any one of claims 13-15 and 18, or the crystal form K of the compound represented by formula (I) according to any one of claims 16-18; and
(b) an optional pharmaceutically acceptable carrier, diluent, or excipient.

26. A preparation method for a pharmaceutical composition, comprising a step of: mixing (a) the crystal form A of the compound represented by formula (I) according to any one of claims 1-3 and 18, the crystal form B of the compound represented by formula (I) according to any one of claims 4-6 and 18, the crystal form C of the compound represented by formula (I) according to any one of claims 7-9 and 18, the crystal form I of the compound represented by formula (I) according to any one of claims 10-12 and 18, the crystal form J of the compound represented by formula (I) according to any one of claims 13-15 and 18, or the crystal form K of the compound represented by formula (I) according to any one of claims 16-18 with (b) an optional pharmaceutically acceptable carrier, diluent, or excipient.

27. Use of the crystal form A of the compound represented by formula (I) according to any one of claims 1-3 and 18, the crystal form B of the compound represented by formula (I) according to any one of claims 4-6 and 18, the crystal form C of the compound represented by formula (I) according to any one of claims 7-9 and 18, the crystal form I of the compound represented by formula (I) according to any one of claims 10-12 and 18, the crystal form J of the compound represented by formula (I) according to any one of claims 13-15 and 18, the crystal form K of the compound represented by formula (I) according to any one of claims 16-18, or the composition according to claim 25 in the manufacture of a medicament for inhibiting KRAS G12D.

28. Use of the crystal form A of the compound represented by formula (I) according to any one of claims 1-3 and 18, the crystal form B of the compound represented by formula (I) according to any one of claims 4-6 and 18, the crystal form C of the compound represented by formula (I) according to any one of claims 7-9 and 18, the crystal form I of the compound represented by formula (I) according to any one of claims 10-12 and 18, the crystal form J of the compound represented by formula (I) according to any one of claims 13-15 and 18, the crystal form K of the compound represented by formula (I) according to any one of claims 16-18, or the composition according to claim 25 in the manufacture of a medicament for treating and/or preventing a disease or disorder, wherein the disease or disorder is a cancer.

29. The use according to claim 28, wherein the disease or disorder is selected from the group consisting of brain cancer, thyroid cancer, head and neck cancer, nasopharyngeal carcinoma, laryngeal cancer, oral cancer, salivary gland cancer, esophageal cancer, gastric cancer, lung cancer, liver cancer, renal cancer, pancreatic cancer, gallbladder cancer, cholangiocarcinoma, colorectal cancer, small intestine cancer, gastrointestinal stromal tumor, urothelial carcinoma, urethral cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, neuroglioma, neuroblastoma, and glioblastoma; preferably, the disease or disorder is selected from the group consisting of pancreatic cancer, colorectal cancer, and non-small cell lung cancer.
